# EUROPEAN PATENT APPLICATION

(11) **EP 1 416 272 A1**
(43) Date of publication of application: **06.05.2004**
(21) Application number: 02738872.7
(22) Date of filing: 28.06.2002
(51) Int. Cl.: G01N 33/50, G01N 33/15, C07K 14/705, C12N 5/00, C07K 16/28, C12Q 1/02, C12Q 1/68, A01K 67/027

(54) **PAEL RECEPTOR, CELLS AND ANIMAL EXPRESSING PAEL RECEPTOR AND METHOD OF SCREENING REMEDY FOR PARKINSON'S DISEASE**

(30) Priority: 28.06.2001 JP 2001197185
(71) Applicant: Riken, Wako-shi, Saitama 351-0198 (JP)
(72) Inventor: TAKAHASHI, Ryosuke c/o RIKEN, Wako-shi, Saitama 351-0198 (JP); IMAI, Yuzuru c/o RIKEN, Wako-shi, Saitama 351-0198 (JP); HATTORI, Nobutaka, Bunkyo-ku, Tokyo 112-0011 (JP); MIZUNO, Yoshikuni, Kita-ku, Tokyo 114-0014 (JP); SODA, Mariko c/o RIKEN, Wako-shi, Saitama 351-0198 (JP); INOUE, Haruhisa c/o RIKEN, Wako-shi, Saitama 351-0198 (JP)
(74) Representative: Woods, Geoffrey Corlett
(86) International application number: PCT/JP2002/006587
(87) International publication number: WO 2003/003010

(57) **Abstract**

An object of the present invention is to provide a substrate of Parkin, a system for elucidating the cause of Parkinson's disease using the substrate and a method of screening for a remedy for Parkinson's disease. An animal cell having a DNA encoding a human Pael receptor or a DNA having a variant of the DNA incorporated therein; a non-human mammal having a DNA carrying the aforementioned DNA incorporated therein; a probe comprising the whole or a part of DNA encoding the human Pael receptor; and an antibody specifically recognizing Pael receptor.

## Description

### TECHNICAL FIELD

The present invention relates to the Pael receptor, which is a substrate of the expression product of the Parkin gene involved in hereditary Parkinson's disease, and an antibody which specifically recognizes the Pael receptor. It further relates to a cell which carries the Pael receptor gene and expresses the Pael receptor, a Parkinson's disease model animal which carries the Pael receptor gene and expresses the Pael receptor, and various experimental methods using them.

### BACKGROUND OF THE INVENTION

Parkinson's disease is the second most common neurodegenerative disorder after Alzheimer's Disease (prevalence rate: 50 to 60 or more people per 100,000 people). Parkinson's disease begins in old age, and is clinically a movement disorder mainly characterized by muscle rigidity, tremors, immobility and the like. The pathological feature of Parkinson's disease is a selective loss of dopamine neurons in the substantia nigra pars compacta of the midbrain. The cause of Parkinson's disease is completely unknown. However, gene mutations have been recently found in cases of familial Parkinson's disease, so that the molecular mechanism is now being elucidated.

Two rare mutations (A53T and A30P) in α-synuclein gene cause autosomal dominant hereditary familial Parkinson's disease (Kruger, R. et al., (1998) Nat Genet 18, 106-108; Polymeropoulos, M. H. et al., (1997) Science 276, 2045-2047). α-synuclein is a presynaptic protein and is a main component of the Lewy body. Lewy bodies are highly ubiquitinated intracellular aggregates, which are pathological features of all the sporadic and a part of familial Parkinson's diseases (Trojanowski, J. Q. et al., (1998) Cell Death Differ 5, 832-837). Lewy bodies are often found in degenerative neurons including dopamine neurons in the substantia nigra, and now are strongly suspected to be involved in the mechanism of the onset of Parkinson's disease (Goldberg, M. S. et al., (2000) Nat Cell Biol 2, E115-119; Spillantini, M. G. et al., (1998) Proc Natl Acad Sci U S A 95, 6469-6473).

Autosomal recessive hereditary juvenile Parkinson's disease (AR-JP, hereinafter referred to as AR-JP in this specification) is the most frequent hereditary Parkinson's disease and is caused by mutations in the Parkin gene. Patients with AR-JP are characterized by Parkinson's-disease-like symptoms resulting from the loss of dopamine neurons without Lewy body formation (Mizuno, Y. et al., (1998) J Neurochem 71, 893-902).

The Parkin gene is 1.5 megabase and is one of the largest human genes. The Parkin gene consists of 12 exons, and encodes a 465-amino acid protein with a molecular weight of 52 kDa (Kitada, T. et al., (1998) Nature 392, 605-608; Shimura, H. et al., (1999) Ann Neurol 45, 668-672). The amino terminal 76 amino acids of Parkin share 62% homology with ubiquitin. There are two RING fingers at the carboxy terminus of Parkin, and a motif resembling RING fingers, which is referred to as "in between RING fingers," is present between the two RING fingers (Morett, E. et al., (1999) Trends Biochem Sci 24, 229-231). Recent studies have revealed that many RING finger proteins have ubiquitin ligase (E3) activity (Jackson, P. K. et al., (2000) Trends Cell Biol 10, 429-439; Joazeiro, C. A. et al., (2000) Cell 102, 549-552). Protein to be degraded by proteasome is modified by covalent binding of ubiquitin. Ubiquitination reaction is performed by 3 types of enzymes: ubiquitin-activating enzyme (E1), ubiquitin-conjugating enzyme (E2) and ubiquitin ligase (E3) (Hershko, A. et al., (1998) Annu Rev Biochem 67, 425-479; Hochstrasser, M. (1996) Annu Rev Genet 30, 405-439). E3 is responsible for promoting ubiquitination by specifically recognizing and binding to the substrate protein. High specificity to recognize a protein to be ubiquitinated is determined by E3, a considerable number of types of which are expected to be present. Multiple groups including our group have revealed that Parkin is E3 and a variant Parkin observed in AR-JP patients lacks E3 activity (Imai, Y. et al., (2000) J Biol Chem 275, 35661-35664; Shimura, H. et al., (2000) Nat Genet 25, 302-305; Zhang, Y. et al., (2000) Proc Natl Acad Sci USA 97, 13354-13359).

### SUMMARY OF THE INVENTION

The onset of AR-JP is caused by dysfunction of Parkin which is E3. Thus it is anticipated that when ubiquitination mediated by Parkin does not occur, the substrate protein remains undegraded and abnormally accumulates intracellularly, so as to induce death in dopamine neurons of the substantia nigra. However, no substrate of Parkin has been discovered so far. Therefore, identification of the substrate proteins of Parkin may be a key to elucidate the pathological mechanism of AR-JP, and to develop a therapy to treat it. Furthermore, dopamine neurons may be specifically degenerated even in the case of sporadic Parkinson's disease under a mechanism shared in common with AR-JP. Thus, it is expected that the identification of the substrates of Parkin can contribute to the elucidation of the causes and the development of therapies for all types of Parkinson's disease.

Hence, an object of the present invention is to provide a substrate of Parkin, a system for elucidating the cause of Parkinson's disease using the substrate and a method of screening for a remedy for Parkinson's disease.

As a result of intensive studies to achieve the above objective, we have completed the present invention related to the Pael receptor by discovering that a protein thought to be a G protein-conjugated membrane protein and named the Pael (Parkin-associated endothelin receptor-like) receptor is the substrate of Parkin, and also by discovering that Pael receptor accumulation is likely to induce dopamine neuronal death in AR-JP.

The present invention is as summarized by the following matters.
(1) A human Pael receptor, for screening for a remedy for Parkinson's disease.
(2) The human Pael receptor of (1) above, wherein Parkinson's disease is autosomal recessive hereditary Parkinson's disease.
(3) An animal cell for screening for a remedy for Parkinson's disease, having a foreign DNA encoding a human Pael receptor or a DNA containing a variant of the DNA incorporated therein.
(4) The animal cell of (3) above, wherein the foreign DNA encoding a human Pael receptor is represented by SEQ ID NO: 1.
(5) The animal cell of (3) or (4) above, further having the Parkin gene or a variant of the Parkin gene.
(6) The animal cell of (5) above further having a gene encoding at least one protein selected from the group consisting of endothelin receptor type A, endothelin receptor type B, UBC6 and UBC7.
(7) The animal cell of any one of (3) to (6) above, wherein the cell is an HEK293T cell or SH-SY5Y cell.
(8) The animal cell of any one of (3) to (7) above, wherein Parkinson's disease is autosomal recessive hereditary Parkinson's disease.
(9) A non-human mammal, having a foreign DNA encoding a human Pael receptor, or a DNA containing a variant of the DNA incorporated therein.
(10) The non-human mammal of (9) above, wherein the foreign DNA encoding a human Pael receptor is represented by SEQ ID NO: 1.
(11) The non-human mammal of (9) or (10) above, further having the Parkin gene or a variant of the Parkin gene.
(12) The non-human mammal of (11) above, further having a gene encoding at least one protein selected from the group consisting of endothelin receptor type A, endothelin receptor type B, UBC6 and UBC7.
(13) The animal of any one of (9) to (12) above, wherein the non-human mammal is a rodent.
(14) The animal of (13) above, wherein the rodent is a mouse.
(15) The non-human mammal of any one of (9) to (14) above, which is a mammal obtained by ontogenesis of a totipotent cell having DNA encoding a human Pael receptor or its variant introduced therein, or the progeny of such mammal, and which carries the above transgene in the somatic cell chromosome.
(16) A cell isolated from the animal of any one of (9) to (15) above.
(17) A method of screening for a remedy for Parkinson's disease, which comprises causing the human Pael receptor of (1) or (2) above to come into contact with a candidate remedy for Parkinson's disease, and using the Pael receptor quantity in animal cells as an indicator.
(18) A method of screening for a remedy for Parkinson's disease, which comprises causing the animal cells of any one of (3) to (8) and (16) above to come into contact with a candidate remedy for Parkinson's disease, and using the Pael receptor quantity in the animal cells as an indicator.
(19) A method of screening for a remedy for Parkinson's disease, which comprises administering a candidate remedy for Parkinson's disease to the animal of any one of (9) to (15) above, and using the Pael receptor quantity in the cell of the animal as an indicator.
(20) The method of any one of (17) to (19) above, wherein Parkinson's disease is autosomal recessive hereditary Parkinson's disease.
(21) A probe, consisting of the whole or a part of a DNA encoding a human Pael receptor for detecting a risk factor for the onset of Parkinson's disease.
(22) The probe of (21) above, wherein Parkinson's disease is autosomal recessive hereditary Parkinson's disease.
(23) A method of detecting a risk factor for the onset of Parkinson's disease, using the probe of (21) or (22) above.
(24) The method of (23) above, wherein Parkinson's disease is autosomal recessive hereditary Parkinson's disease.
(25) An antibody recognizing the Pael receptor, which reacts with the Pael receptor but does not react with ETBR-LP-2 which is a Pael receptor homolog.
(26) The antibody of (25) above, which further does not react with endothelin receptor type A or endothelin receptor type B, which is a Pael receptor homolog.
(27) The antibody of (25) or (26) above, which is a monoclonal antibody. The present invention is described in detail as follows.

### 1. Obtainment of DNA encoding the Pael receptor

DNA encoding the Pael receptor can be obtained according to a method described in a publication well known by a person skilled in the art, such as J. Sambrook, E. F. Fritsch & T. Maniatis (1989), Molecular Cloning, a laboratory manual, second edition, Cold Spring Harbor Laboratory Press, or Ed Harlow and David Lanc (1988) Antibodies, a laboratory manual, Cold Spring Harbor Laboratory Press. For example, the DNA can be obtained as the substrate of Parkin by constructing cDNA library from RNA extracted from the brain of a human, and then screening the library according to the yeast two-hybrid method using Parkin as bait. Here the term "Parkin" means the expression product of the Parkin gene involved in hereditary Parkinson's disease. The DNA can also be obtained by screening the library using an oligonucleotide probe synthesized based on the DNA sequence encoding the Pael receptor.

Examples of a DNA encoding the Pael receptor include a DNA containing the nucleotide sequence represented by SEQ ID NO: 1, and a DNA encoding the amino acid sequence represented by SEQ ID NO: 2.

A variant of the DNA encoding the Pael receptor can be prepared by inducing mutations in the cDNA of a wild type Pael receptor by a known method. Examples of a variant of the DNA encoding Pael receptor include a DNA which hybridizes under stringent conditions to a DNA containing the nucleotide sequence represented by SEQ ID NO: 1 and encodes a protein that can be the substrate of Parkin, or a protein which contains an amino acid sequence derived from the amino acid sequence represented by SEQ ID NO: 2 by deletion, substitution or addition of one or a plurality of amino acids and can be the substrate of Parkin. Here the term "stringent conditions" mean conditions wherein a so-called specific hybrid is formed, but non-specific hybrids are not formed. Under an example of such conditions, DNAs having high homology, specifically, DNAs having homology of 60% or more, or preferably 80% or more hybridize to each other, whereby nucleic acids sharing low homology do not hybridize to each other. More specifically, the stringent conditions comprise a sodium concentration of 150 to 900 mM or preferably 600 to 900 mM, and a temperature of 60 to 68°C or preferably 65°C.

As an amino acid sequence that is derived from the amino acid sequence represented by SEQ ID NO: 2 by deletion, substitution or addition of one or a plurality of amino acids, for example, an amino acid sequence may be derived from the amino acid sequence of SEQ ID NO: 2 by the deletion of 1 to 10, preferably 1 to 5, and more preferably 1 or 2 amino acids, or by the substitution of 1 to 10, preferably 1 to 5, and more preferably 1 or 2 amino acids with other amino acids. Moreover, 1 to 10, preferably 1 to 5, and more preferably 1 or 2 amino acids may be added to the amino acid sequence represented by SEQ ID NO: 2.

An amino acid sequence derived from the amino acid sequence represented by SEQ ID NO: 2 by deletion, substitution or addition of one or a plurality of amino acids includes, for example, at least 60% or more, preferably 80% or more, and further preferably 95% or more homology with the amino acid sequence of SEQ ID NO: 2, when calculated by BLAST.

Once the nucleotide sequence of a gene is determined, a DNA encoding the Pael receptor or a variant thereof can then be obtained by chemical synthesis, PCR using the cloned cDNA as a template, or hybridization using a DNA fragment having the nucleotide sequence as a probe. Thus by the use of the obtained DNA, a protein that contains an amino acid sequence derived from the amino acid sequence of SEQ ID NO: 2 by deletion, substitution or addition of one or a plurality of amino acids and can be the substrate of Parkin can be obtained.

Furthermore, the DNA encoding a human Pael receptor of the present invention includes a DNA containing not only the ORF portion of the Pael gene but also the 5' untranslated region and the 3' untranslated region.

Here, the term "substrate of Parkin" means a protein that is Parkin-dependently ubiquitinated and degraded by in the presence of ATP, E1, E2 and ubiquitin. For example, whether or not a protein is the substrate of Parkin can be confirmed by mixing the protein *in vitro* with ATP, E1, E2 and ubiquitin, and then confirming if it is ubiquitinated and degraded.

Furthermore, gene diagnosis can be performed using the whole or a part of the DNA encoding the Pael receptor, so that whether or not the gene carries a risk factor for the onset of Parkinson's disease can be diagnosed. At this time, the length of a DNA sequence to be used ranges from 12 nucleotides to 16 nucleotides or more, and is preferably 20 nucleotides or more. Moreover, polynucleotides having these sequences and nucleotides that are capable of hybridizing under the above stringent conditions to the polynucleotides can also be used for diagnosis.

### 2. Obtainment of recombinant Pael receptor-expressing cell and Pael receptor

DNA encoding the Pael receptor of the present invention represented by SEQ ID NO: 1 and the variant thereof are inserted to an expression vector, and then the vector is introduced into an appropriate host cell, so as to be able to obtain Pael receptor-expressing cells. Any vector such as a plasmid, phage, or virus can be used as long as it is replicable in host cells. Examples of a vector include *Escherichia coli* plasmids such as pBR322, pBR325, pUC118, pUC119, pKC30 and pCFM536, *Bacillus subtilis* plasmids such as pUB110, yeast plasmids such as pG-1, YEp13 and YCp50, and phage DNA such as λgt110 and λZAPII. Examples of a vector for mammalian cells include virus DNA such as baculovirus, vaccinia virus and adenovirus, and SV40 and the derivative thereof. The vector contains the replication initiation point, a selection marker and a promoter, and may also contain an enhancer, a transcription termination sequence (terminator), a ribosome binding site, or a polyadenylation signal and the like, if necessary. Any promoter may be used, as long as it is effectively expressed in host cells. Examples of a promoter include an SRα promoter, an SV40 promoter, an LTR promoter, a CMV promoter and an HSV-TK promoter. In addition, when a protein is expressed on the membrane surface, a nucleic acid encoding a known signal peptide may be added to the N-terminus.

Examples of a host cell include bacterial cells such as *Escherichia coli, Streptomyces* or *Bacillus subtilis*, Fungi cells such as strains of the genus *Aspergillus,* yeast cells such as baker's yeast or methanol-assimilating yeast, insect cells such as those of *Drosophila* S2 or *Spodoptera* Sf9, and mammalian cells such as HEK293T, SH-SY5Y, CHO, COS, BHK, 3T3 or C127.

Transformation can be performed by a known method such as calcium chloride, calcium phosphate, DEAE-dextran-mediated transfection or electroporation.

Pael receptors can be isolated from the thus obtained Pael receptor-expressing cells.

Furthermore, animal cells expressing Pael receptors can be used in screening for an agent that can be used for treating Parkinson's disease such as a substance that suppresses Pael receptor accumulation, a substance that promotes Pael receptor ubiquitination or degradation, or a substance that promotes Pael receptor discharge outside the body.

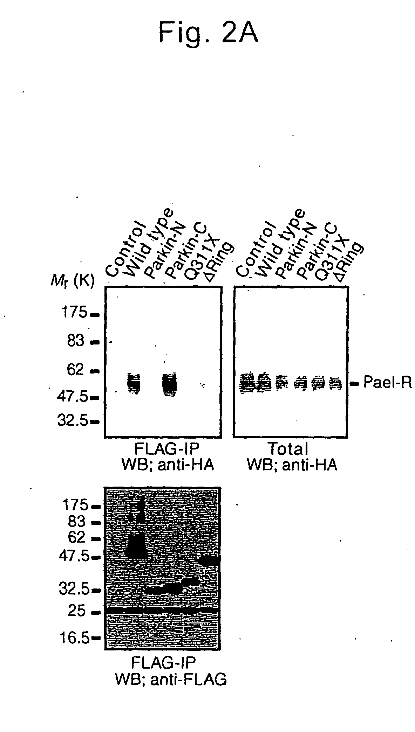

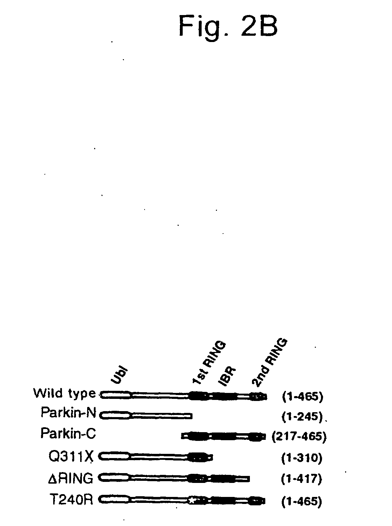

Furthermore, not only can screening for an agent that can be used for treating Parkinson's disease be performed, but also a cell line that can be used for elucidating the cause of Parkinson's disease can be obtained by the introduction and co-expression of a gene encoding the Pael receptor with the Parkin gene (Kitada, T. et al., (1998) Nature 392, 605-608; Shimura, H, et al., (1999) Ann Neurol 45, 668-672), a variant of the Parkin gene, a gene encoding Pael-receptor-associated receptor such as an endothelin receptor type A or type B (Arai, H et al.(1990) Nature 348, 730-732; Sakurai, T et al. (1990) Nature 348, 732-735), and/or an endoplasmic reticulum-associated E2 gene (Katsanis N, Fisher EM (1998) Genomics 51, 128-31; Lester D, et al. (2000) Biochem Biophys Res Commun 269, 474-80). Here, a variant of the Parkin gene means a gene whose expression product lacks the biological action of Parkin such as E3 activity or the like. Examples of a variant of the Parkin gene include a Parkin gene isolated from an AR-JP patient (Imai, Y. et al., (2000) J Biol Chem 275, 35661-35664; Shimura, H. et al., (2000) Nat Genet 25, 302-305; Zhang, Y. et al., (2000) Proc Natl Acad Sci USA 97, 13354-13359), and Parkin-N, Q311X or ΔRING losing the C-terminal amino acid sequence of Parkin (Fig. 2).

### 3. Obtainment of anti-Pael receptor antibody

Anti-Pael receptor antibodies can be prepared by inoculating animals such as mice, guinea pigs, rabbits or goats with Pael receptors as antigens subcutaneously, intramuscularly, intraperitoneally or intravenously several times according to a method well known by a person skilled in the art for sufficient immunization, collecting blood from the animals, and performing serum separation. At this time, an appropriate adjuvant can also be used. A monoclonal antibody can also be prepared by a known method. For example, monoclonal antibodies can be prepared by fusing splenocytes of a mouse immunized with the Pael receptor with mouse myeloma cells to produce hybridomas, administering intraperitoneally the culture supernatant of the hybridomas or the hybridomas to mice, and then preparing from the mouse ascites. The Pael receptor to be used as an antigen for immunization may be a natural protein extracted from brain tissue, a recombinant protein, or a chemically-synthesized protein. Furthermore, the Pael receptor may also be a protein having the entire amino acid sequence, or a peptide fragment having a partial structure of the protein and a fusion protein with another protein. A peptide fragment that can be used herein may be a fragment obtained by degrading the protein with an appropriate protease, or may be a product expressed by incorporating the whole or a part of the nucleotide sequence represented by SEQ ID NO: 1 into an expression vector. A polypeptide fragment that is chemically bound with an appropriate carrier protein may also be used. The reactivity of the thus obtained antibody can be measured by a method well known by a person skilled in the art such as enzyme immunoassay (EIA), radioimmunoassay (RIA) or Western blotting. At this time, by the use of a homolog of the Pael receptor such as LP-2 as an antigen to be used for the assay of reactivity in addition to the Pael receptor, a specific antibody that recognizes the Pael receptor but does not recognize the homolog of the Pael receptor such as LP-2 can be obtained.

### 4. Obtainment of Pael receptor-expressing model animal

A promoter sequence or an enhancer sequence to regulate expression is ligated to a gene to be introduced into a Pael receptor-expressing model animal. These sequences are not specifically limited, and appropriate combinations of sequences that are generally employed can be used. To specifically express a transgene in the brain, β-actin promoter or the like is preferably used.

Transgenic animals can be generated by, for example, introducing the above transgene into totipotent cells of a mammal according to the method of Pro. Natl. Acad. Sci. USA 77:7380-7384, 1980 or another method, allowing the cells to develop into individual animals, and then selecting individual animals carrying the transgene incorporated in the genome of the somatic cells. A preferred mammal is a mouse, of which many inbred lines have been produced, and for which techniques for culturing the fertilized eggs, for external fertilization and the like have been well established. However, technically, any animal species can be a target. Examples of a totipotent cell, into which a gene is introduced, include, in the case of a mouse, culture cells such as ES cells having pluripotency, in addition to fertilized eggs and early embryos. As a method of introducing a gene into a culture cell, for example, a known electrostatic pulse method, the liposome method or the calcium phosphate method can be used. In terms of generation efficiency regarding individual transgenic animals and transmission efficiency regarding the transmission of a transgene to the next generation, a physical method (microinjection) of injecting a DNA solution into a fertilized egg is preferred.

A totipotent cell into which a gene has been injected is transplanted in the fallopian tubes of a pseudo parent, and then the cell is allowed to develop into an individual animal, so that a Pael receptor-expressing animal can be obtained. DNA is extracted from the somatic cell, and then the presence of the transgene can be confirmed by Southern blot analysis, PCR assay or the like. By the use of animals confirmed to carry the transgene as Founders for breeding, animals wherein Pael receptor gene or a variant thereof has been stably incorporated into a part of the chromosome can be efficiently generated.

Furthermore, similar to the above Pael receptor-expressing cells, the Parkin gene, a variant of the Parkin gene isolated from an AR-JP patient, and/or a gene encoding a Pael-receptor-associated receptor such as the endothelin receptor may be incorporated simultaneously.

The thus generated transgenic animal can be an optimum model animal for elucidating the cause of Parkinson's disease and screening for an agent that can be used for treating Parkinson's disease.

Furthermore, transgenic animals provided by the present invention carry transgenes in all of their somatic cells, so that cells isolated from the individual animal also express Pael receptors. Hence, the culture system of these cells can also be utilized for elucidating the cause of Parkinson's disease and screening for an agent that can be used for treating Parkinson's disease, similarly to the case of the above individual animals.

### 5. Screening for agent that can be used for treating Parkinson's disease

The above Pael receptor, Pael receptor-expressing cells, transgenic animals and the cells obtained from the transgenic animal can be used for screening for an agent that can be used for treating Parkinson's disease.

Examples of an agent that can be used for treating Parkinson's disease include a substance that suppresses Pael receptor accumulation, a substance that promotes Pael receptor ubiquitination or degradation, and a substance that promotes Pael receptor discharge outside the body.

Applicability of a candidate substance as an agent can be determined by adding the candidate substance as an agent that can be used for treating Parkinson's disease to Pael receptor-expressing culture cells, or administering it to Pael receptor-expressing animals, and then measuring the quantity or the ubiquitination degree of the Pael receptor within the cell or within the animal body. Thus, screening for a remedy can be performed.

### BRIEF DESCRIPTION OF DRAWINGS

FIG.1 shows the binding of Parkin with the Pael receptor.
FIG.2A shows the binding of Parkin with the Pael receptor and that of a Parkin variant with the Pael receptor.
FIG.2B shows Parkin and a Parkin variant used for an experiment to examine the binding of Parkin with the Pael receptor and that of a Parkin variant with the Pael receptor.
FIG.3 shows the binding of endogenous Parkin with the endogenous Pael receptor in the brain tissue and in the culture cell.
FIG.4 shows the results of examining Pael receptor ubiquitination.
FIG.5 shows the results of examining the binding of Parkin with endoplasmic reticulum-associated E2.
FIG.6 shows the result of *in vitro* ubiquitination assay of the Pael receptor using endogenous Parkin derived from the culture cell.
FIG.7 shows the result of *in vitro* ubiquitination assay using recombinant Parkin and E2.
FIG.8 shows the result of *in vitro* ubiquitination assay using the Pael receptor and its homolog as a substrate.
FIG.9A shows the results of detection by radioautography in the pulse-chase experiment to confirm whether or not Parkin degrades the Pael receptor in the ubiquitin-proteasome system.
FIG.9B shows the results of quantifying with phosphoimagin in the pulse-chase experiment to confirm whether or not Parkin degrades the Pael receptor in the ubiquitin-proteasome system.
FIG.10 shows that Pael receptors easily initiate misfolding and are insolubilized by endoplasmic reticulum stress.
FIG.11 shows the result of examining the effect of a proteasome inhibitor and that of an UPR inducer on cell death resulting from the overexpression of Pael receptors.
FIG.12 is a photograph showing the morphology of the dead cells expressing Pael receptors.
FIG.13 shows photographs showing immunolocalization of Pael receptors over-expressed by SH-SY5Y cells.
FIG.14 shows photographs showing intracellular inclusion bodies of Pael receptors.
FIG.15 shows how Parkin suppresses cell death resulting from misfolded Pael receptors.
FIG.16 is a graph showing how Parkin suppresses cell death resulting from misfolded Pael receptors.
FIG.17A shows the results of Western blotting to detect endogenous Pael receptor quantity in the human brain.
FIG.17B shows the relative value of endogenous Pael receptor quantity in the human brain.

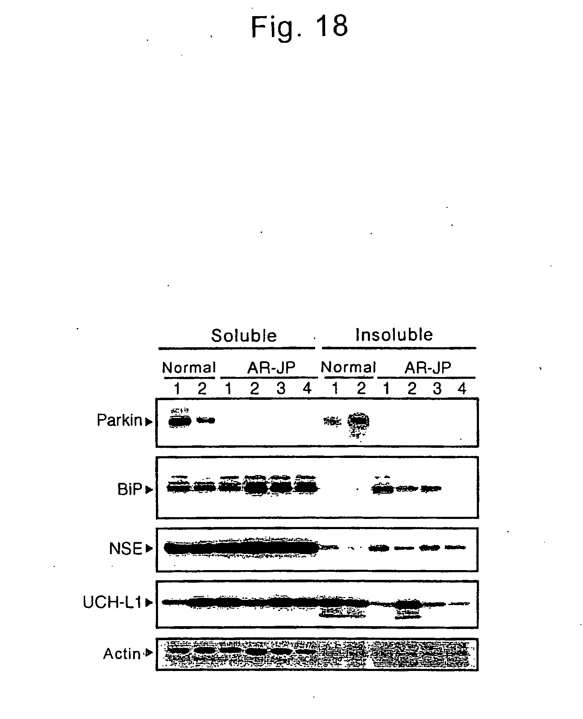

FIG. 18 shows the results of Western blot analyses of total protein derived from 20 µg of soluble or 10 µg of insoluble fractions.

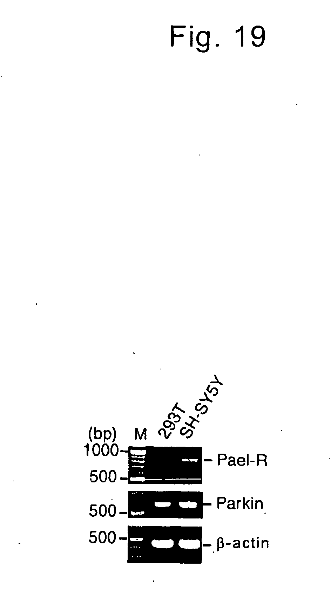

FIG.19 shows the results of RT-PCR using total RNA extracted from SH-SY5Y cells and 293T cells as templates.
FIG.20 shows the specificity of the anti-Pael receptor antibody.
FIG.21 shows photographs showing Pael receptor expression in the brain.

### BEST MODE FOR CARRYING OUT THE INVENTION

The present invention will be described more specifically by the following examples. These examples are not intended to limit the present invention.

In the examples of the present invention, plasmids, antibodies and culture cells were prepared as follows.

Descriptions concerning expression plasmids for Parkin, Parkin variants, UBCH6, UBCH7 and Ubiquitin are as described in Imai, Y. et al. (2000) J Biol Chem 275, 35661-35664. The full-length complementary strand DNAs (cDNAs) of human and mouse Pael receptors, human ETBR-LP-2, human UBC6, UBC7 and E2-25K were obtained by reverse transcription (RT)-PCR. The full-length cDNA of ETA-R and that of ETB-R were obtained from the RIKEN Gene Bank. Recombinant GST-Parkin, GST-Δ Ex 4, 6xHis tagged UBC6 and UBC7 were produced in the *Escherichia coli* BL21 strain (DE3) pLysS (Novagen). Site-directed mutagenesis to the active center cysteine of UBC6 and UBC7 was performed by the PCR method. UBC6 of a putative membrane insertion site-deficient type (UBC6ΔC; 1 to 288 amino residues) is expressed better than wild type UBC6 in *Escherichia coli* and has high enzyme activity, so that UBC6ΔC was subjected to *in vitro* ubiquitination assay. Anti-Myc (9E10), anti-HA (Y-11), anti-Ubiquitin (FL-76), anti-BiP (N-20) and anti-actin (C-2) antibodies were purchased from Santa Cruz Biotech. Anti-FLAG (M2), anti-HA (3F10), anti-Ubiquitin (1B3), anti-GluR4 (AB 1508) and anti-NSE (BBS/NC/VI-H14) were purchased from Sigma, Roche Diagnostics, MBL (Nagoya, Japan), Chemicon and Dako, respectively. Anti-UCH-L1 antibodies were provided by Dr. Keiji WADA (National Center of Neurology and Psychiatry, Japan). 293T cells derived from a human fetal kidney and SH-SY5Y cells derived from neuroblastoma were subjected to gene transfer, immunoprecipitation, Western blot, immunocytochemistry and cell death assay according to the method as described in Imai et al., 2000.

Yeast two-hybrid screening, immunoprecipitation and Western blot analysis, immunochemical analysis, a pulse-chase experiment, *in vitro* ubiquitination assay and RT-PCR were performed by the following methods.

### [Yeast two-hybrid screening]

Plasmid pGBT9 (Clontech) with cDNA of human full-length Parkin inserted therein was prepared as bait for library screening. Yeast two-hybrid screening was performed for a mixture of the cDNA library (Clontech), which is of the human adult whole brain and inserted in pACT2, and the cDNA library (produced in our laboratory), which is of the human midbrain substantia nigra and inserted in pGAD424. Library screening was performed according to the instructions of a Matchmaker Two-Hybrid System Kit (Clontech).

### [Immunoprecipitation and Western blot analysis]

Cells were suspended in a cytolysis buffer (consisting of 20 mM HEPES, pH 7.4, 120 mM NaCl, 5 mM EDTA, 10% glycerol, 1% Triton X-100 and Complete Protease Inhibitors [Roche Diagnostics]) and then lysed at 4°C for 30 minutes. The forebrain cortex of the human cerebrum was suspended in a cytolysis buffer, and then crushed using a downs homogenizer. The sample was fractionated into surfactant-soluble and -insoluble fractions, and then immuno-precipitated according to a modified method of Ward et aI. (1995) Cell 83, 121-127. Specifically, the suspension was fractionated by centrifugation at 15,000×g for 30 minutes. The supernatant derived from 1% Triton X-100 soluble fraction was directly subjected to immunoprecipitation. The insoluble pellet fraction was washed 4 times in an ice-cooled cytolysis buffer. Subsequently, the fraction was subjected to lysis at 60°C for 1 hour in a cytolysis buffer supplemented with sodium lauryl sulfate (SDS) with a final concentration of 1%. A cytolysis buffer containing 10 mM MgCl₂ and 25 µg/ml DNase I and having a volume 10 times greater than that of the solution was added. The solution was allowed to react at 37°C for 10 minutes. After centrifugation at 15,000×g for 30 minutes, the supernatant derived from 1% Triton X-100 insoluble fraction was subjected to immunoprecipitation using various antibodies and protein G-coupled or protein A/G (50/50%)-coupled Sepharose beads (Amersham-Pharmacia). The immunoprecipitate was washed 4 times in a cytolysis buffer containing no complete protease inhibitors. 1% Triton X-100-soluble and -insoluble fractions of total cell extract, or immunoprecipitates derived from the 1% Triton X-100-soluble fraction and -insoluble fraction were subjected to Western blot analysis using ECL detection reagent (Amersham-Pharmacia). Proteins were quantified using Coomassie protein assay reagent (Pierce).

### [Immunochemistry]

For immunocytochemistry, a human Pael receptor expression vector (1 µg per sample) was gene-transferred, or was gene-transferred in combination with 0.3 µg of red fluorescent protein expression vector (pDsRed1-N1, Clontech) having endoplasmic reticulum localization sequence of calreticulin or Golgi body localization sequence of human β1, 4-galactosyltransferase into SH-SY5Y cells inoculated on a 8-well chamber slide. After 20 hours of culturing, the cells were exposed to 20 µM lactacystin for 16 hours. After washed in phosphate buffered saline, the cells were fixed with 0.2% glutaraldehyde and 2% formaldehyde. Subsequently, the cells were stained with anti-human Pael receptor antibodies or anti-BiP antibodies. For immunohistochemistry, the carboxyl terminal site (541 to 600 amino residues) of mouse Pael receptor was expressed as a GST fusion protein in *Escherichia coli.* The anti-Pael receptor polyclonal antibody was prepared against a recombinant protein from which the GST portion had been removed. 14 µm-thick frozen sections of the mouse C57BL/6 brain tissue were allowed to react with anti-Pael receptor antibodies (1:200 dilution), anti-TH antibodies (Chemicon, 1:100), anti-NeuN antibodies (Chemicon, 1:200) or anti-CNPase antibodies (Sigma, 1:100). The primary antibody was stained with Alexa 488 or Alexa 546 (Molecular Probes)-labeled secondary antibody. The stained cells or sections were mounted using SlowFade (Molecular probes), and then observed under a confocal laser fluorescence microscope (Fluoview, Olympus).

### [Pulse-chase experiment]

Pael receptors having FLAG-tagged carboxyl termini (Pael receptor-FLAG) and expression plasmids for Parkin or control plasmids were gene-transferred to SH-SY5Y cells. 36 hours later, the cells were cultured for 1 hour in Dulbecco's modified eagle's medium (M/C-free DMEM) containing 5% fetal calf serum (FCS) but containing no methionine/cysteine under conditions involving the presence or the absence of 10 µM lactacystin. The cells were then labeled at 37°C for 30 minutes by adding 200 µCi/ml ³⁵S-methionine/cysteine to the 5% FCS-containing M/C-free DMEM in the presence or the absence of 10 µM lactacystin. The cells were then washed and cultured for 3 hours in 10% FCS-containing Dulbecco's modified eagle's medium under conditions involving the presence or the absence of 10 µM lactacystin. After each time of chasing, the cells were lysed, subjected to immunoprecipitation using anti-FLAG M2 affinity gel (Sigma), separated by SDS-polyacrylamide gel electrophoresis, and then visualized using an image analyzer (BAS-5000; Fujifilm). The metabolically labeled Pael receptor was quantified using Image Gauge software (Fujifilm).

### [In vitro ubiquitination assay]

³⁵S-labeled Pael receptor-FLAG was produced using TNT quick coupled transcription/translation systems (Promega) under conditions involving the presence or the absence of canine pancreatic microsomal membranes (Promega), and then immunoprecipitated using anti-FLAG M2 affinity gel. Both Pael receptors produced under conditions involving the presence or the absence of microsomal membranes could be used without any difference as substrates for *in vitro* ubiquitination assay. Hence, Pael receptors produced under conditions involving the absence of microsomal membranes were used for most assays. *In vitro* ubiquitination assay was performed according to the method described in Imai Y. et al. (2000) J Biol Chem 275, 35661-35664, except for using 167 pmol of ubiquitin (Sigma).

### [RT-PCR]

Total RNA was extracted using ISOGEN reagent (NIPPON GENE, Japan, Toyama). Single-stranded cDNA was synthesized from 5 µg of total RNA using the SuperScript First Strand Synthesis kit (GIBCO (present name: Invitrogen)). Oligo-dT-primed cDNA was amplified by the PCR method. Primers used for PCR are as follows: Pael receptor forward primer, 5'-CCTCCAGCTCTTCCTTCAGA-3' ; Pael receptor reverse primer, 5'-TTTCTGCCGGAGCTCGGCCA-3' ; Parkin forward primer, 5'-GGAGGCGACGACCCCAGAAAC-3' ; Parkin reverse primer, 5'-GGGACAGCCAGCCACACAAGG-3' ; β-actin forward primer, 5'-CAAGGCCAACCGCGAGAAGA-3' ; and β-actin reverse primer, 5'-GGAAGGCTGGAAGAGTGCCT-3'. The quality of RNA was confirmed with the messenger RNA of β-actin.

### [Example 1] Isolation of gene encoding Pael receptor

A human brain cDNA library was subjected to screening by the yeast two-hybrid method using Parkin as bait to identify the substrate of Parkin. It was shown by BLAST database search that a positive clone obtained by screening of 8,500,000 independent clones encodes a part of a homolog of endothelin receptor type B that is of the G-protein-conjugated type (Donohue, P. J. et al., (1998) Brain Res Mol Brain Res 54, 152-160; Marazziti, D. et al., (1997) Genomics 45, 68-77; Zeng, Z. et al., (1997) Biochem Biophys Res Commun 233, 559-567). This receptor was newly named the Pael (Parkin-associated endothelin receptor-like) receptor.

### [Example 2] Preparation of anti-Pael receptor antibody

The carboxyl terminal site (541 to 600 amino residues) of the mouse Pael receptor was expressed in *Escherichia coli* as a GST fusion protein. The anti-Pael receptor polyclonal antibody was produced against a recombinant protein from which the GST portion had been removed.

Anti-Parkin monoclonal antibody was produced against recombinant 6x His-tagged human Parkin protein expressed by *Escherichia coli*. Anti-human Pael receptor monoclonal antibody was produced using as an antigen 293T cells that had been caused to over-express the human Pael receptor.

### [Example 3] Preparation of Pael-receptor-expressing cell and examination of the interaction between Pael receptor and Parkin

Expression plasmids encoding C-terminal FLAG-tagged Pael receptors or similarly tagged Pael-receptor-associated receptors (endothelin receptor type A and type B) were temporarily transfected with expression plasmids for human Parkin to HEK293T cells, so as to cause the expression of the receptors.

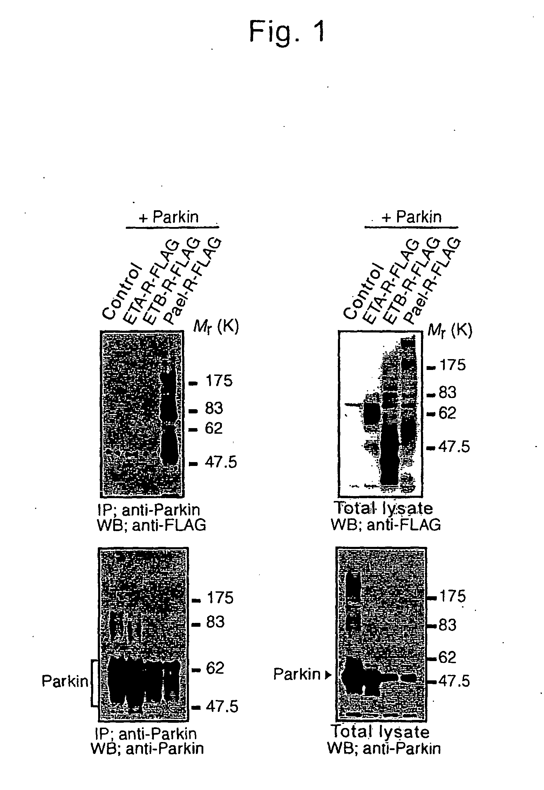

Vectors containing no gene inserted therein (Control), FLAG-tagged Pael receptors (Pael receptor-FLAG), FLAG-tagged endothelin receptor type A or B (ETA-R- or ETB-R-FLAG) and Parkin genes were inserted in plasmids. The plasmids were gene-transferred to 293T cells. 293T cells were then lysed in a cytolytic solution, and then the solution was immunoprecipitated using anti-Parkin polyclonal antibodies. Immunoprecipitates (IP) and soluble total cell extract (Total lysate) were analyzed by Western blotting (WB). Figure 1 shows the results. It was confirmed that the Pael receptor was specifically, and the endothelin receptor type B was very weakly, immunoprecipitated with Parkin (Fig. 1, upper panel).

In addition, when the Pael receptor or the endothelin receptor type B was over-expressed by 293T cells, and then Western blotting was performed, these proteins appeared in the form of smear and high-molecular-weight bands. These results suggest that these proteins are modified by saccharification, ubiquitination or the like. In addition, a high proportion of the cells expressing Pael receptors or endothelin receptor type B died (40 % and 20%, respectively), and their expression levels of Parkin were low (Fig. 1, lower panel).

### [Example 4] Examination of the binding site of Parkin with Pael receptor

Next, the region of Parkin to which the Pael receptor binds within a mammalian cell was examined. A series of variants of Parkin and wild type Parkin were co-expressed as FLAG-tagged proteins with HA-tagged Pael receptors by 293T cells, and then co-precipitated. Haemagglutinin (HA)-tagged Pael receptor, a vector (Control) having no gene inserted therein, FLAG-tagged Parkin (Wild type), Parkin-N, Parkin-C, Q311X or ΔRING gene was inserted into a plasmid. The plasmid was gene-transferred to 293T cells, and then the cells were lysed in a cytolytic solution. The solution was then immunoprecipitated with anti-FLAG monoclonal antibodies (FLAG-IP), and then analyzed by Western blotting (WB). Figure 2 shows the results of examining the binding of Parkin with the Pael receptor and that of Parkin variant with the Pael receptor. The C-terminal portion (217 to 465: amino acid portion) of Parkin and the full-length Parkin were efficiently co-precipitated to the same degree with the Pael receptor. However, in the case of Parkin having a mutation in the C-terminal portion, the efficacy of co-precipitation was lowered or such Parkin and the receptor were not co-precipitated (Fig. 2). The schematic view (lower right) shows Parkin and Parkin variant used for the identification of the binding region of the Pael receptor. Numbers in a parenthesis correspond to each amino residue encoding Parkin protein. An asterisk (*) indicates the site of point mutation. UbI indicates ubiquitin-like region. RING indicates RING-finger motif. IBR indicates in-between-RING-finger. These results revealed that the C-terminal portion of Parkin is essential for the binding with the Pael receptor.

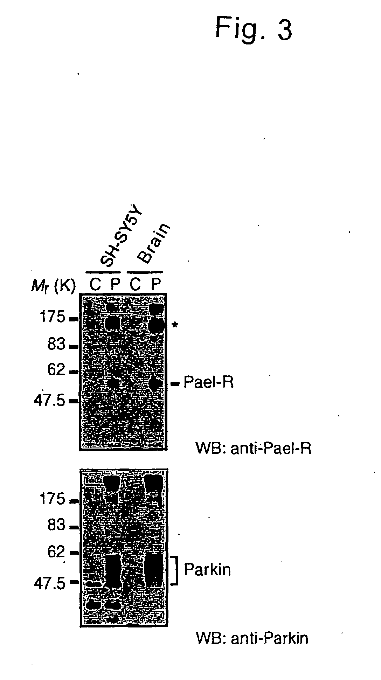

Furthermore, physiological binding of endogenous Parkin with the Pael receptor was checked using dopaminergic neuroblastoma SH-SY5Y cells and the human brain tissue (both expressing endogenous Pael receptor mRNA). The human brain or neuroblastoma-derived SH-SY5Y cells were dissolved by the above method, and then the supernatant fraction was imunoprecipitated (IP) with preimmune sera (C) or anti-Parkin antisera (P). The co-precipitated Pael receptors were detected by Western blotting (WB) using anti-Pael receptor monoclonal antibodies. Fig. 3 shows the results of examining the binding of endogenous Parkin and the endogenous Pael receptor in the brain tissue and the culture cells. As shown in Fig. 3, the immunopositive band of the Pael receptor was co-precipitated with endogenous Parkin. In the figure, an asterisk (*) indicates the band of the reactivity of the anti-Pael receptor antibody of approximately 120 kDa. This band is often observed *in vivo,* and is thought to show an unknown state of the Pael receptor.

The examination results described in Examples 2 to 4 using Pael receptor-expressing cells revealed that Parkin binds to the Pael receptor but the Parkin variant does not bind to the Pael receptor.

### [Example 5] Pael receptor Ubiquitination by Parkin in Pael receptor-expressing cell

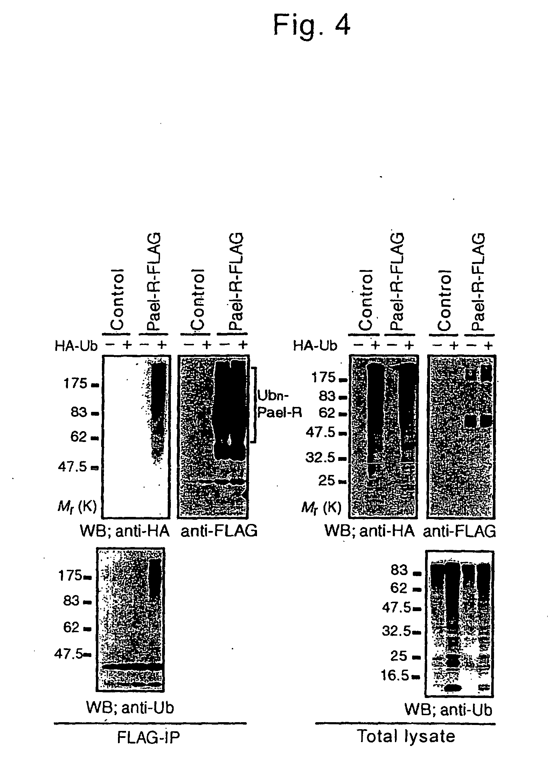

Next, since Pael receptors over-expressed by culture cells were observed to have higher molecular weights as shown in Fig. 1 when Western blotting was performed, whether or not the Pael receptors were ubiquitinated was examined. HA-tagged ubiquitin (HA-Ub) was over-expressed with FLAG-tagged Pael receptors by 293T cells, followed by immunoprecipitation with anti-FLAG antibodies. Vectors (Control) having no gene inserted therein or Pael receptor-FLAG were gene-transferred to SH-SY5Y cells, in combination with HA-tagged Ub (HA-Ub) or the vector only (-). Immunoprecipitates (FLAG-IP) that had been immunoprecipitated with anti-FLAG monoclonal antibodies and a soluble total cell extract (Total lysate) were analyzed by Western blotting (WB) using anti-FLAG, anti-HA or anti-Ub antibodies. The immunoprecipitated Pael receptors were ubiquitinated with HA-Ub. Without further expression of HA-Ub, a band of the Pael receptors having a higher molecular weight was recognized by the use of anti-ubiquitin antibodies (Fig. 4). These facts suggest that a Pael receptor protein fails to fold normally, so that it is degraded as a protein having an abnormal structure by the ubiquitin-proteasome degradation system.

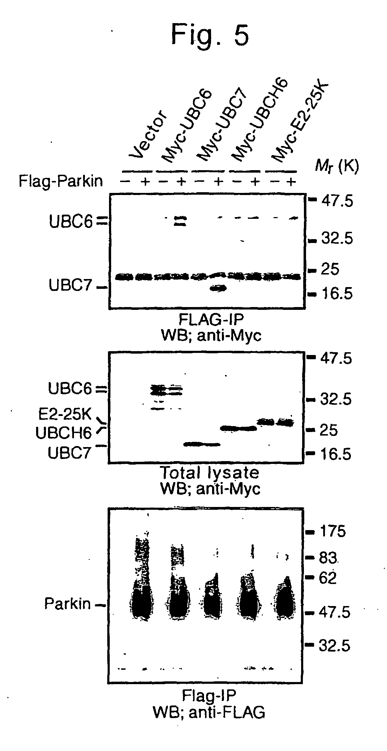

We have previously reported that the expression of Parkin is increased due to the stress induced by misfolded proteins, so as to have an effect of suppressing cell death resulting from the stress induced by misfolded proteins (Imai, Y. et al. (2000) J Biol Chem 275, 35661-35664). Against endoplasmic reticulum stress, a series of unfolded protein responses (UPR) including endoplasmic reticulum (ER)-associated degradation (ERAD), which regulate gene expression, are induced (Travers, K. J. et al., (2000) Cell 101, 249-258). ERAD degrades misfolded membrane proteins or secretory proteins with the degradation system within the cytoplasm (Plemper, R. K. et al., (1999) Trends Biochem Sci 24, 266-270). ERAD substrate was transported back through the membrane of the endoplasmic reticulum to the cytoplasm, in which it is ubiquitinated and degraded by the action of UBC6, UBC7, proteasome complex and the like (Biederer, T. et al., (1997) Science 278, 1806-1809; Bordallo, J. et al., (1998) Mol Biol Cell 9, 209-222; Gilon, T. et al., (2000) Mol Cell Biol 20, 7214-7219; Wilhovsky, S. et al., (2000) Mol Biol Cell 11, 1697-1708). These data suggest that Parkin is E3 of the RING type whose expression is increased by UPR and a possible involvement of Parkin in ERAD. Thus, the binding of Parkin and endoplasmic reticulum-associated E2 was checked. Various E2 plasmids tagged with Myc were co-expressed with FLAG-tagged Parkin by human 293T cells and then co-precipitation was attempted. Vectors having no gene inserted therein or FLAG-Parkin cDNA were gene-transferred to 293T cells, in combination with the vector only (-), Myc-tagged UBC6 (Myc-UBC6), Myc-UBC7, Myc-UBCH6 or Myc-E2-25K. Immunoprecipitates (FLAG-IP) immunoprecipitated with anti-FLAG monoclonal antibodies and soluble total cell extract (Total lysate) were analyzed by Western blotting (WB) using anti-FLAG or anti-Myc antibodies. As a result, it was shown that Parkin binds to endoplasmic reticulum-associated E2, UBC6 and UBC7, but does not bind ihtracellularly to E2 (UbcH6, E2-25K) used as a control (Fig. 5).

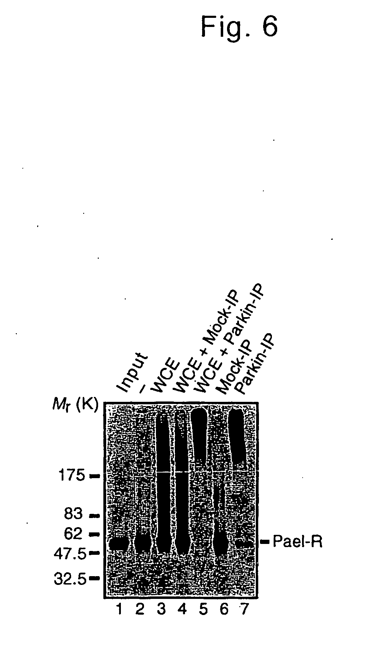

Furthermore, whether or not Parkin can ubiquitinate the Pael receptor *in vitro* in cooperation with endoplasmic reticulum-associated E2 was evaluated. To reproduce the ubiquitination reaction of the Pael receptor *in vitro,* Parkin immunoprecipitated from SH-SY5Y cells or a simple extract of SH-SY5Y cells was added with the pure products of Ub and E1. In the presence of Ub and E1, ³⁵S-labeled Pael receptor-FLAG was allowed to react with the following factors added: WCE, the soluble total cell extract of SH-SY5Y; Mock-IP, the immunoprecipitate obtained from SH-SY5Y cells using preimmune serum; and Parkin-IP, the immunoprecipitate obtained from SH-SY5Y cells using anti-Parkin anti-serum. As a result, it was shown that the immunoprecipitate of Parkin promotes Pael receptor ubiquitination far more significantly than a simple cell extract (Fig. 6). In Fig. 6, "Input" and "-" indicate Pael receptors before reaction and Pael receptors that were allowed to react in the presence of only Ub and E 1 for 90 minutes, respectively. It is remarkable that the immunoprecipitate of Parkin ubiquitinated Pael receptors even when E2 was not added (Fig. 6, lanes 6 and 7).

These results suggest that immunologically-isolated Parkin mediates the transfer of ubiquitin from E2 of cells, which have formed complexes with Parkin within the cells, to the Pael receptor.

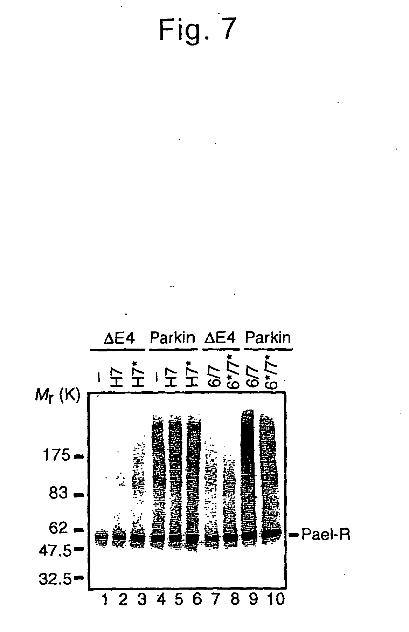

To test the possibility, ubiquitination assay was performed *in vitro* using the fusion protein, GST-Parkin and GST-ΔE4 as E3 that was a fusion protein of exon 4-deficient Parkin (observed in AR-JP patients) with GST produced by *Escherichia coli.* It was predicted that the Pael receptor is a type of membrane protein to be incorporated, and a nascent Pael receptor having an abnormal structure would be degraded by ERAD. Thus, in addition to UbcH7 that is known to function as E2 of Parkin, UBC6 (or UBC6ΔC which was obtained by removing the hydrophobic carboxyl terminal region to be embedded within the membrane from UBC6; 1 to 288 amino acids) which is endoplasmic reticulum-associated E2 and UBC7 were used in this assay. In the presence of Ub and E1 similar to the above examination, ³⁵S-labeled Pael receptor-FLAG was caused to react with combinations of recombinant E2 (H7, UBCH7; 6/7, UBC6ΔC and UBC7), GST-fused Parkin and GST-fused exon4-deficient variant Parkin. As shown in Fig. 7, GST-Parkin significantly promoted Pael receptor ubiquitination although no E2 was added, suggesting that endogenous E2 had previously bound to transcribed/translated Pael receptors in the lysate of reticulocyte (Fig. 7). In the figure, an asterisk (*) indicates that cysteine of the active center of E2 was substituted with serine. In contrast, the recombinant protein of deficient variant Parkin had no action to ubiquitinate Pael receptors even when E2 was added (Fig. 7, lanes 1 to 3, 7 and 8). A combination of UBC6ΔC, UBC7 and Parkin significantly enhanced ubiquitination. However, when inactive variants of UBC6ΔC and UBC7 were used instead, Pael receptor ubiquitination was attenuated to the standard level (Fig. 7; comparison was made among lanes 4, 9 and 10).

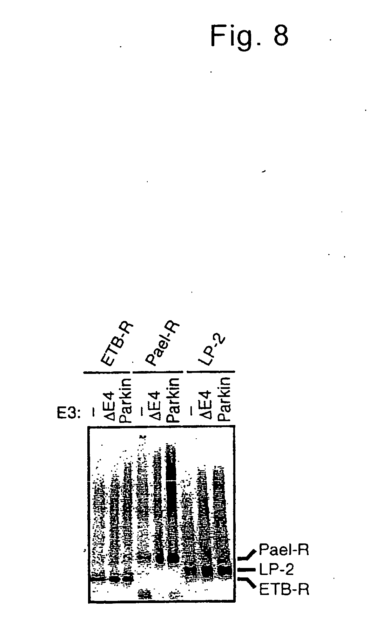

Furthermore, ³⁵S-labeled Pael receptor, ETB-R or ETBR-LP-2 (LP-2) was allowed to react in the presence of UBC6ΔC and UBC7, similarly to the above examination. Ubiquitination reaction that occurred with the combination of recombinant UBC6 (or, UBC6 Δ C), UBC7 and GST-Parkin was extremely specific to Pael receptors, and ETB-R and ETBR-LP-2, which were molecules closely related to the Pael receptor, were not ubiquitinated (Fig. 8). Together with the fact (Fig. 5) that Parkin binds to UBC6/7, the *in vitro* ubiquitination assay suggests that UBC6 and UBC7 are involved together with Parkin in the degradation system of Pael receptors.

### [Example 6] Degradation of Pael receptor by Parkin

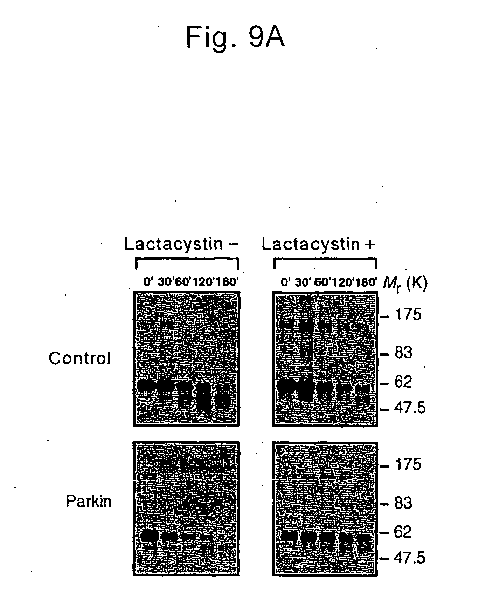

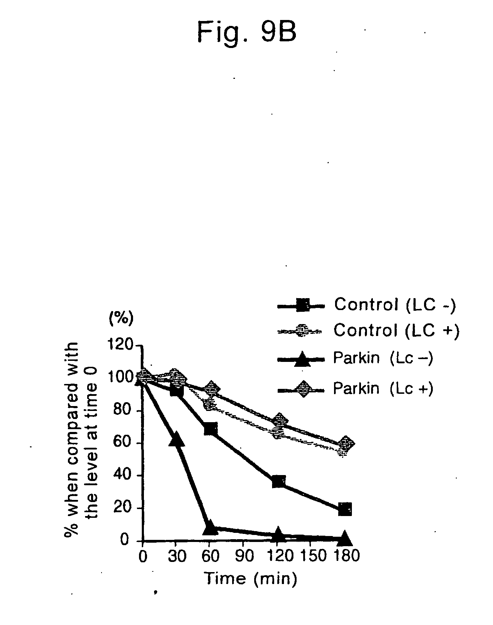

Next, to examine whether Parkin degrades the Pael receptor in the ubiquitin-proteasome system, the turnover rate of the Pael receptor was measured under conditions involving the presence or the absence of Parkin by the pulse-chase experiment (Fig. 9). Expression plasmids for Pael receptor-FLAG were gene-transferred to SH-SY5Y cells, in combination with vectors (Control) having no gene inserted therein or FLAG-Parkin expression plasmids. The cells were then cultured under conditions involving the presence or the absence of 10 µM lactacystin. Subsequently, ³⁵S-methionine/cysteine were added for labeling, and then the cells were chased for the time indicated in Fig. 9 in the presence (Lc +) or the absence (Lc -) of 10 µM lactacystin. ³⁵S-labeld Pael receptors were immunoprecipitated, detected by radioautography (Fig. 9 left), and then quantified by phosphor imaging (Fig. 9 right). The levels of the labeled Pael receptors were plotted by comparing the level at time 0 (min). In the absence of lactacystin, the proteasome inhibitor, 18% of nascent Pael receptors remained in the control cells after 180 minutes of chasing. Lactacystin caused the degradation rate of Pael receptors to decrease also in control cells and cells over-expressing Parkin. Specifically, 54% of Pael receptors and 58% of the same remained after 180 minutes in the control cells and the cells over-expressing Parkin, respectively. In contrast, degradation of Pael receptors was significantly promoted in the cells over-expressing Parkin, so that after 180 minutes only 0.8% of Pael receptors remained. These results show that Parkin degrades the Pael receptor through the ubiquitin-proteasome degradation system.

### [Example 7] Accumulation of Pael receptor in Pael receptor-expressing cell

When over-expressed by culture cells, Pael receptors were highly ubiquitinated even in the absence of a proteasome inhibitor (Fig. 4). This finding reminds us of findings concerning CFTR or the δ opioid receptor that are also highly ubiquitinated. 75% of nascent CFTR and 60% of nascent δ opioid receptors misfold in the endoplasmic reticulum (Jensen, T. J. et al., (1995) Cell 83, 129-135; Petaja-Repo, U. E. et al., (2001) J Biol Chem 276, 4416-4423; and Ward, C. L. et al., (1995) Cell 83, 121-127).

Such proteins are transported back from the endoplasmic reticulum through' the translocon (a specific channel in the endoplasmic reticulum) composed of Sec61p and the like to the cytoplasm (Plemper, R. K. et al., (1999) Trends Biochem Sci 24, 266-270). These proteins are subsequently treated by the endoplasmic reticulum associated degradation (ERAD) system depending on the ubiquitin-proteasome system of the cytoplasm.

When cells are exposed to a proteasome inhibitor, accumulation of CFTR molecules that are insoluble to a non-ionic detergent and have been highly ubiquitinated is increased. Because of this fact, it was examined to find what quantity of Pael receptors that are soluble or insoluble in a detergent was present.

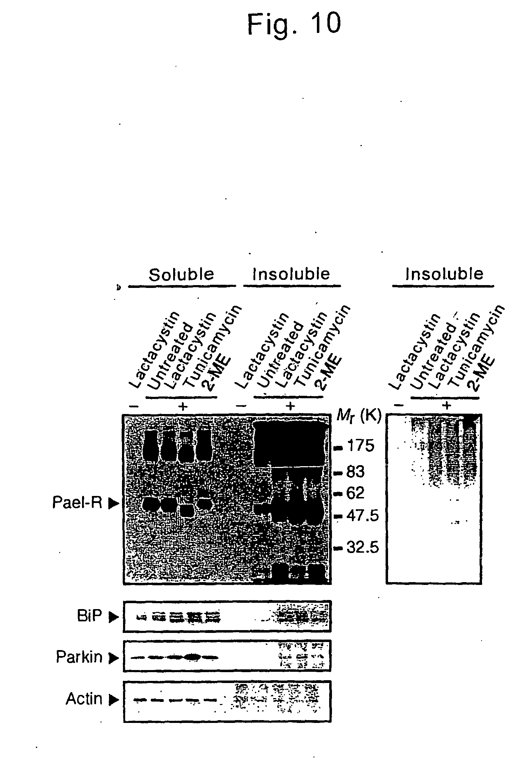

Vectors (-) having no gene inserted therein or expression plasmids (+) for Pael receptors were gene-transferred to SH-SY5Y cells, and then the cells were cultured for 20 hours. Next, in the presence or the absence of lactacystin (10 µM) and in the presence of tunicamycin (1 µg/ml) or 2-mercaptoethanol (2-ME; 1 mM), the cells were cultured for 16 hours. The cells were lysed in a buffer containing 1% Triton X-100, and then fractionated by the method described in Ward et al., 1995 Cell 83, 121-127. Each fraction was Western-blotted using an antibody against a protein shown in Fig. 10. Insoluble Pael receptors were immunoprecipitated as described above from the insoluble fraction of the same sample, and then analyzed using anti-Ub polyclonal antibodies (Fig. 10). 1% Triton-X100-insoluble or -soluble fraction was obtained under various conditions, including cases wherein Pael receptor cDNA had not been transfected (-) or transfected (+) to SH-SY5Y cells, and then subjected to Western blotting. Even in an untreated state, Pael receptors were already present in the insoluble fraction at a level equivalent to that of the soluble fraction.

Furthermore, Pael receptors in the fraction, which were insoluble in the presence of lactacystin (proteasome inhibitor), or tunicamycin (an inhibitor of saccharification) or 2-ME (reducing agent) that causes UPR, increased with a high-molecular-weight shift. Subsequently, insoluble Pael receptors were extracted from the insoluble fraction of the same sample, and then the presence or the absence of ubiquitination was examined using antibodies. In proportion to Pael receptor accumulation in the insoluble fraction, high-molecular-weight bands resulting from ubiquitination were detected, when Western blotting was performed using anti-ubiquitin antibodies for the immunoprecipitates of Pael receptors from the insoluble fraction. In contrast, such high-molecular-weight bands were not observed in samples untreated with any agent. Furthermore, it was revealed that the addition of lactacystin or an agent (tunicamycin or 2-ME) that induces UPR to the cells over-expressing Pael receptors caused increases in the expression of BiP (GRP78) of ER chaperone and that of endogenous Parkin in the soluble fraction, and also caused the same in the insoluble fraction to increase to some extent (Fig. 10).

These results agree with our past findings that a UPR inducer increases the expression of Parkin and that of BiP (Imai, Y. et al. (2000) J Biol Chem 275, 35661-35664).

Based on the facts that Pael receptors were highly ubiquitinated (Fig. 4) and the increased expression of BiP was observed when insoluble Pael receptors were accumulated (Fig. 10), it was predicted that cell death specifically caused by Pael receptor accumulation would be cell death induced by misfolded proteins.

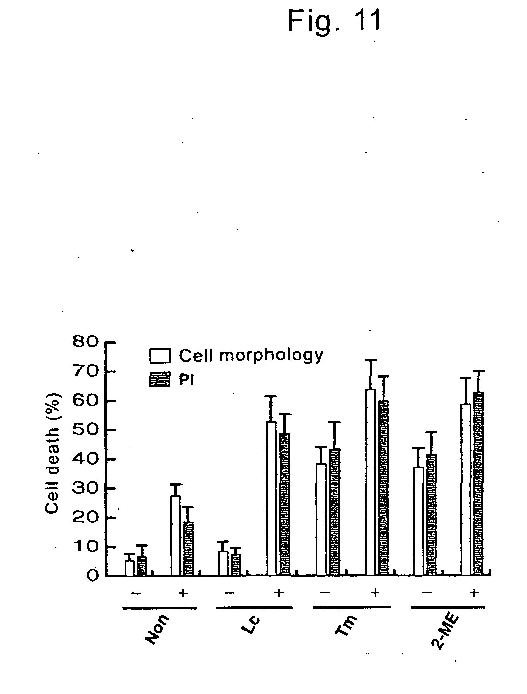

To confirm this prediction, the effect of a proteasome inhibitor and that of a UPR inducer on cell death resulting from the overexpression of Pael receptors were examined (Fig. 11). Vectors (-) having no gene inserted therein or expression plasmids for Pael receptors (+) were gene-transferred with expression plasmids for enhanced green fluorescent proteins (EGFP) to SH-SY5Y cells. The cells were then cultured in the presence (Lc, 20 µM) or the absence (non) of lactacysin, and the presence of tunicamycin (Tm, 5 µg/ml) or 2-ME (2 mM) for 16 hours. Cell death was counted by the method described in Imai, Y. et al. (2000) J Biol Chem 275, 35661-35664. In Fig. 11, the line of error indicates the standard deviation (S.D.) calculated from three experiments.

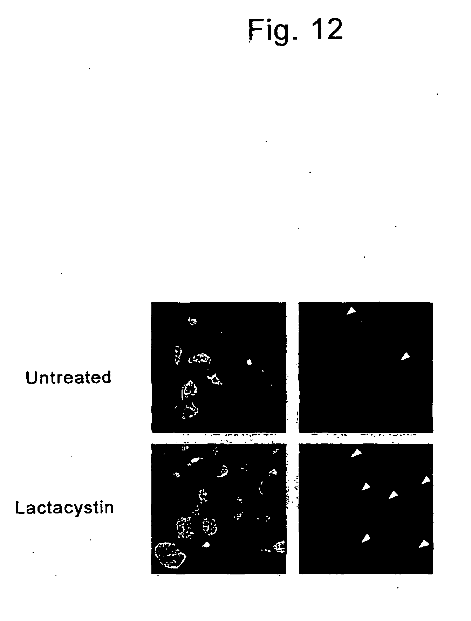

Approximately 20% of the cells died because of the overexpression of Pael receptors. Even when SH-SY5Y cells were treated with 20 µM lactacystin for 16 hours, cell death did not particularly increase compared with the case of untreated cells. In contrast, 40% of even control cells died because of the presence of tunicamycin (5 µg/ml) or 2-ME (2 mM), the UPR inducer. Under the same conditions, approximately 50% of Pael receptor-over-expressing cells died in the presence of lactacystin (Figs. 11 and 12). Fig. 12 shows the morphology of dead cells expressing Pael receptors. Expression plasmids for Pael receptors were gene-transferred to SH-SY5Y cells, and then the cells were cultured in the presence or the absence of lactacystin. The cells expressing Pael receptors were visualized (green, cells observed white in top and bottom panels on the left photographs) using anti-Pael receptor monoclonal antibodies. The cell nuclei were contrast-stained with 4', 6-diamidino-2-phenylindole (cells observed blue or gray). Dead cells that expressed Pael receptors and initiated chromatic agglutination are indicated by arrowheads.

Similarly, approximately 60% of the cells died due to tunicamycin or 2-ME (Fig. 11).

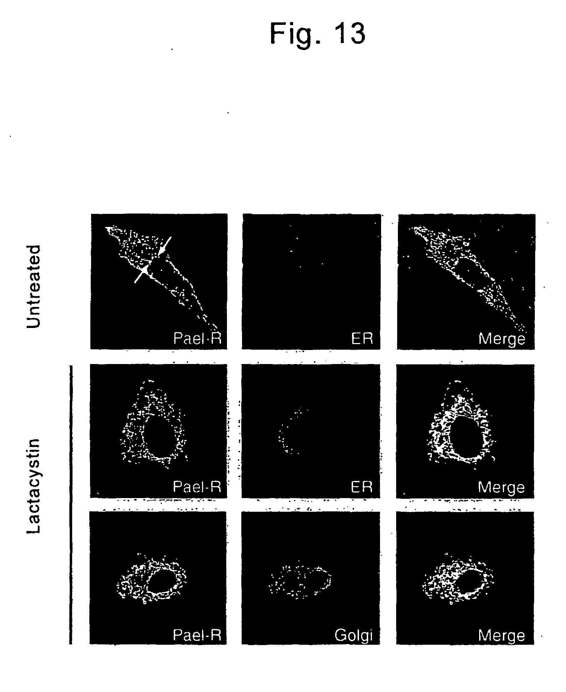

Furthermore, to confirm the involvement of cell death induced by misfolded proteins in cell death resulting from overexpression of Pael receptors, intracellular distribution of Pael receptors in the presence or the absence of lactacystin was examined. Figure 13 shows the results. Figure 13 shows the immunolocalization of Pael receptors over-expressed by SH-SY5Y cells. Expression plasmids for Pael receptors were gene-transferred to SH-SY5Y cells, and then the cells were cultured in the presence (middle and lower panels) or the absence (upper panel) of lactacystin for 6 hours. Cells expressing Pael receptors were visualized using anti-Pael receptor monoclonal antibodies (green, portions observed white in the upper, middle and lower rows on the left line). The endoplasmic reticulum and the Golgi body were respectively counter stained using anti-BiP antibodies and DsRed-Golgi (red, portions observed black in the upper, middle and lower rows on the middle line). Yellow indicates that the localization of Pael receptors completely coincided with that in the endoplasmic reticulum rather than that in Golgi body (Portions observed white in the upper, middle and lower rows on the right line. The middle line, which shows the result of counter staining with the endoplasmic reticulum, was all observed white, however, there are portions stained with colors other than white in the upper and lower rows). The arrow indicates Pael receptors accumulated around the nucleus.

SH-SY5Y cells having Pael receptor plasmids transfected therein were cultured under conditions of lactacystin (+) or (-), and then immunocytochemistry was performed using an antibody against the Pael receptor or BiP. Although some of Pael receptors were observed around the nuclei or in the cytoplasm in consistent with the past report, Pael receptors were mainly expressed on the untreated SH-SY5Y cell surfaces. Thus, the Pael receptor was thought to be a cell membrane protein. In contrast, when the cells were treated with lactacystin for 6 hours, Pael receptors were accumulated in the endoplasmic reticulum so as to interfere with the expression on the cell membranes. Accumulation of Pael receptors in the endoplasmic reticulum was confirmed by co-staining with anti-BiP antibodies, and using pDsRed-ER, which was a plasmid encoding a red fluorescent protein having the endoplasmic reticulum targeting signal (data not shown). The specific distribution of Pael receptors not in the Golgi body but in the endoplasmic reticulum was further confirmed by co-staining with WGA labeled with red fluorescent protein (DsRed-Golgi) having a Golgi body-targeting signal or rhodamine.

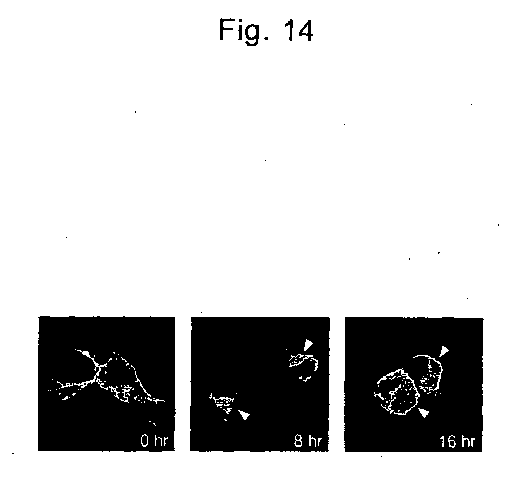

The above results were the same as those obtained by treatment with tunicamycin or 2-ME (data not shown). In fact, when culture was continued for 8 to 16 hours in the presence of such an agent, inclusion bodies resembling aggresomes appeared adjacent to the nuclei. Figure 14 shows intracellular inclusion bodies of the Pael receptor. At this time, the cell body came to have a smaller round shape and seemed to be about to die. SH-SY5Y cells wherein expression plasmids for Pael receptor had been gene-transferred were cultured in the presence of 20 µM lactacystin for a time as indicated. Localization of Pael receptors in the cell was visualized using anti-Pael receptor monoclonal antibodies (green, portions observed white (after staining) in the photographs). Inclusion bodies of Pael receptors around the nucleus are indicated by arrowheads.

Taken together with the prior results of Western blotting, and as shown by the results of the immunocytochemical experiments that the accumulation of over-expressed Pael receptors into the endoplasmic reticulum involves an increase in the expression of BiP, cell death may be finally induced by endoplasmic reticulum stress induced by misfolded proteins (Kozutsumi, Y. et al., (1988) Nature 332, 462-464).

### [Example 8] Suppression by Parkin of Pael receptor accumulation and cell death in Pael receptor-expressing cells

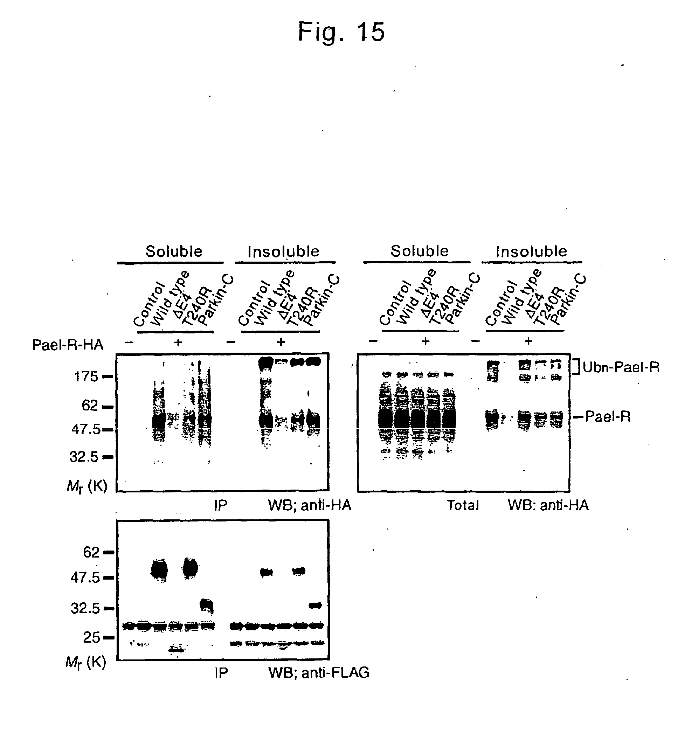

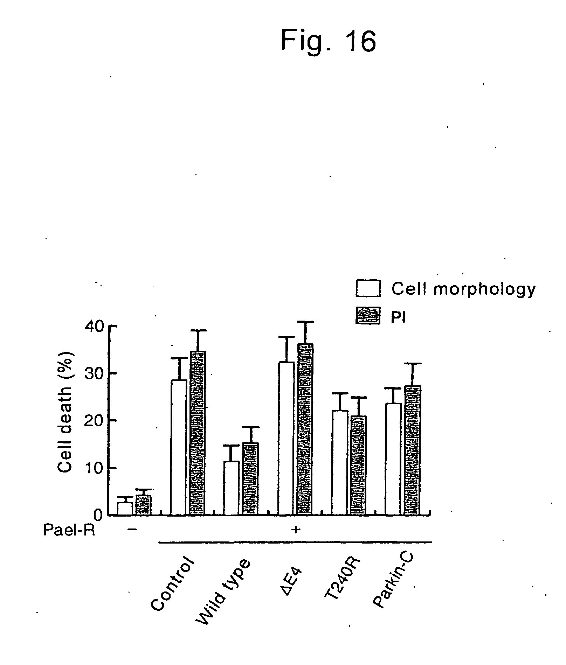

Based on the fact that Parkin suppresses cell death induced by misfolded proteins, it was predicted that the overexpression of Parkin would rescue cells from cell death resulting from the overexpression of Pael receptors. FLAG-Parkin and a series of variants of FLAG-Parkin were transfected together with vector plasmids or Pael receptors to SH-SY5Y cells. Extracts of the transfected cells were divided into 1% Triton-soluble and -insoluble fractions. Fractions were then immunoprecipitated using anti-FLAG antibodies, and then the immunoprecipitates were analyzed by Western blotting. Vectors (-) having no gene inserted therein or expression plasmids (+) for Pael receptor-HA were gene-transferred to SH-SY5Y cells, in combination with control vectors (Control) or FLAG-Parkin (Wild type), FLAG-ΔE4, FLAG-T240R or FLAG-Parkin-C expression plasmids. The SH-SY5Y cells were solubilized, and then the products were divided into 1% Triton X-100-soluble (S) or 1% Triton X-100-insoluble (I) fractions, followed by immunoprecipitation using anti-FLAG monoclonal antibodies (IP). Co-precipitated Pael receptors were detected by Western blotting using anti-HA monoclonal antibodies. Poly-ubiquitinated Pael receptors were confirmed by Western blotting the same membrane using anti-Ub antibodies (data not published), and were termed Ubₙ-Pael receptors. Furthermore, combinations of expression plasmids were gene-transferred with EGFP expression plasmids as a reporter to SH-SY5Y cells, and then the cells were cultured for 48 hours. Cell death was counted by a method similar to that employed for the examination whose results are shown in Fig. 11. Figures 15 and 16 show the results.

In agreement with the analytical results shown in Figs. 1 to 3, soluble Pael receptors were specifically co-precipitated by immunoprecipitation with Parkin, Parkin-C, and partially a variant carrying a point mutation (T240R), of the soluble fractions. Also in the results obtained with the insoluble fraction, co-precipitation of Pael receptors was confirmed with Parkin, Parkin-C and partially a variant carrying a point mutation (T240R). Pael receptors co-precipitated with FLAG-Parkin or a variant thereof appeared in the form of a smear band showing a high-molecular-weight shift, suggesting that the receptors were ubiquitinated. The Pael receptor quantity in the insoluble fraction significantly decreased in the presence of wild type Parkin. In contrast, the effect of Parkin to decrease insoluble Pael receptors partially or completely disappeared in the variant Parkin, specifically, T240R, Parkin-C or ΔE4. In agreement with the data of insoluble Pael receptors, cell death resulting from overexpression of Pael receptors was suppressed significantly by wild type Parkin and partially suppressed by T240R or Parkin-C variant, but was not suppressed at all by ΔE4 (Fig. 16). These results revealed that wild type Parkin degrades Pael receptors through the action of proteasme before the receptors are accumulated in an insoluble fraction, but variant Parkin does not have such action.

### [Example 9] Measurement of Pael receptor accumulation using anti-Pael receptor antibody

Based on the above results, it was concluded that specific dopamine cell death in AR-JP is likely induced by Pael receptor accumulation, the substrate of Parkin. To obtain direct evidence to support the conclusion, the quantity of endogenous Pael receptors in human brain was measured.

Autopsy brains of 4 AR-JP patients and 2 control patients were homogenized, and then divided into 1% Triton X-100-soluble and -insoluble fractions. These fractions were further immunoprecipitated using monoclonal antibodies against Pael receptors or control antibodies. Subsequently the quantity of the immuno-precipitated Pael receptors was standardized using the quantity of immunoprecipitates of GluR4, the glutamate receptor.

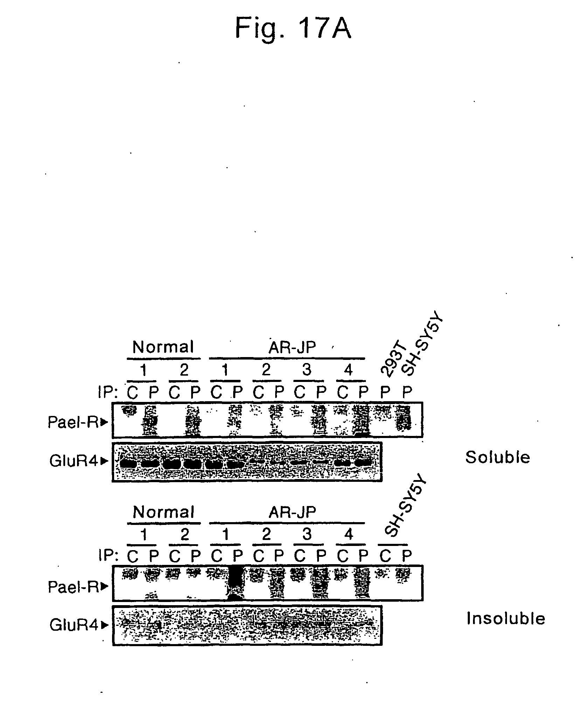

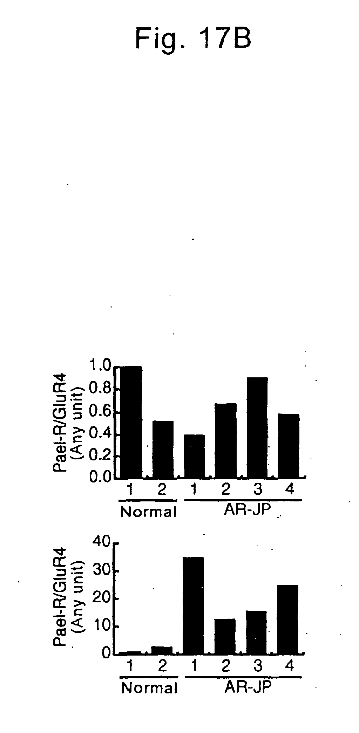

AR-JP and the anterior lobe cortex of a human cerebrum (control), SH-SY5Y cells and 293T cells were divided into 1% Triton X-100-soluble and -insoluble fractions. The fractions were then immunoprecipitated (IP) using anti-Pael receptor monoclonal antibodies (clone 1, P) or control IgG (mouse IgG2b, C) of the same isotype. Subsequently, each fraction was immunoprecipitated using anti-GluR4 monoclonal antibodies. The immunoprecipitates were analyzed by Western blotting using another anti-Pael receptor monoclonal antibody (clone 7) or anti-GluR4 monoclonal antibody. The immunoprecipitates obtained using anti-Pael receptor monoclonal antibodies were quantified by Quantity One software (BioRAD). The results were standardized against the immunoprecipitate obtained using anti-GluR4 monoclonal antibodies in the corresponding lane (Fig. 17).

The Pael receptor level obtained by immunoprecipitation from the soluble fraction of AR-JP sample was 0.4 to 1.0 times greater than that of the control sample (Normal 1). There was no difference between the two. However, in the case of the insoluble fraction, the Pael receptor in the case of AR-JP brain increased to a level 12 to 35 times greater than that in the case of the control brain (Normal 1). Both soluble and insoluble fractions were prepared from SH-SY5Y cells and 293T cells (293T cells have Parkin mRNA but do not have Pael receptor mRNA), and then similarly analyzed. The same band was observed in the immunoprecipitate of Pael receptors of the insoluble fraction of SH-SY5Y cells, but not observed in the immunoprecipitate of Pael receptors of the soluble fraction of 293T cells. This is a result that certifies the specificity of the antibody against the Pael receptor.

Similar to the case of the normal brain homogenate, an extremely small quantity of Pael receptors was extracted from the insoluble fraction of SH-SY5Y cells. This suggests that when the expression level of Pael receptors is at a normal level, insoluble Pael receptors are not accumulated. Furthermore, ubiquitinated Pael receptors were not observed in either normal brain or AR-JP brain, even when the immunoprecipitates of Pael receptors were examined using anti-ubiquitin antibodies.

It was supposed that Pael receptor accumulation would be caused by Parkin deficiency, and the relationship between Pael receptor accumulation and the resulting UPR was examined. The soluble and insoluble fractions of control and AR-JP brain samples were examined by Western blotting using anti-BiP antibodies. BiP increased in most soluble fractions of AR-JP brain compared with the control. Moreover, BiP was observed in most insoluble fractions of AR-JP brain. These results suggest that UPR is induced in AR-JP brain, and misfolded proteins, to which BiP bind, are present in the insoluble fractions. By Western blotting using anti-NSE, anti-UCH-L1 and anti-actin antibodies, it was found that there was no significant difference in the quantity of the proteins derived from neurons and glia cells in the samples.

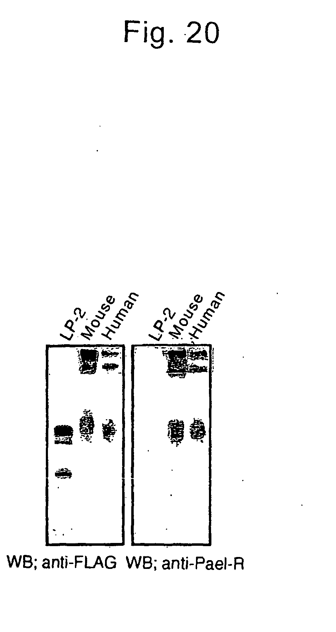

Furthermore, it was checked whether or not Pael receptors were expressed by dopamine neurons (of the substantia nigra pars compacta), which are specifically degenerated and died in Parkinson's disease. First an anti-serum against the Pael receptor was prepared. This anti-serum is an antibody with particularly high specificity such that it recognizes mouse and human Pael receptors, but does not recognize LP-2, ETA-R or ETB-R, the homolog of the Pael receptor. Figure 20 shows the specificity of anti-Pael receptor antibodies. The extract of 293T cells that over-expressed mouse Pael receptor-FLAG, human Pael receptor-FLAG or ETB-LP-2-FLAG (LP-2) was analyzed by Western blotting using anti-FLAG antibodies (left) or anti-Pael receptor antibodies (right).

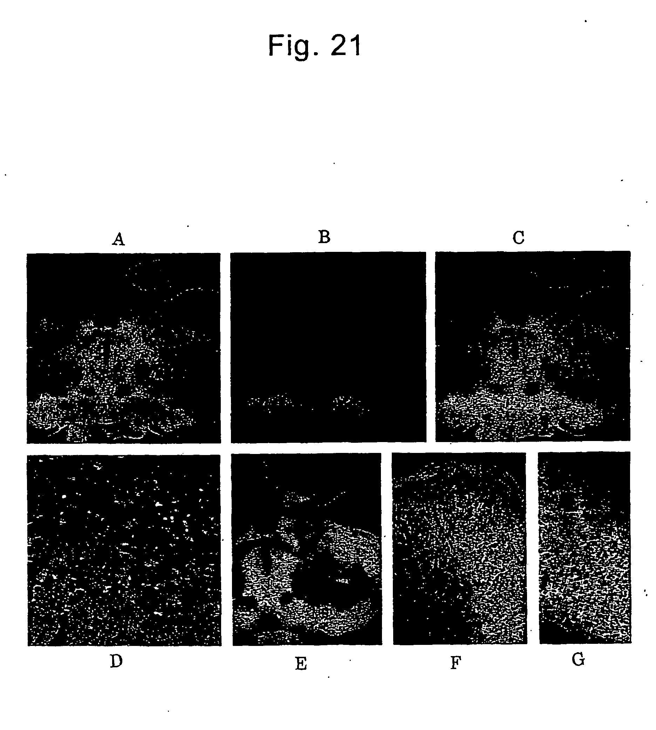

Figure 21 shows the results of immunohistochemical analysis. A to D in Fig. 21 show the immunolocalization of Pael receptors (A, green, portions observed white (after staining) in Fig. 21 A) and tyrosine hydroxylase (B, red, portions observed white (after staining) in Fig. 21B) in a section thinly sliced along the vertex of the mouse brain. Yellow indicates that tyrosine hydroxylase-positive neurons expressed Pael receptors in the substantia nigra pars compacta (C and D, Portions particularly observed white (after staining) in Fig. 21C, and overlapping the portions stained white in Fig. 21B; portions observed white (after staining) in Fig. 21 D). Original amplification of (A-C) was "x 40" or that of (D) was "x 400." E to G in Fig. 21 indicate that Pael receptors were mainly expressed by oligodendrocytes in the brain. E and G: in serial sections (A to D), Yellow indicates that CNPase positive cells (red) expressed (green) Pael receptors. F and G: localization of Pael receptors (green) and NeuN (F, red) or CNPase (G, red) in the cerebral cortex is shown. Original amplification of (E) was "x 40," or that of (F or G) was "x 100."

Immunohistochemically, Pael receptors were broadly expressed in the brain, including the midbrain substantia nigra (Fig. 21 A). Most Pael receptors were CNPase-positive oligodendrocytes (Fig. 21 E and G). On the other hand, most NeuroN-positive cells, that is, neurons, had no Pael receptor or expressed the receptor only weakly (Fig. 21F). However, it was shown that most tyrosine hydroxylase-positive cells were Pael receptor-positive, and dopamine neurons of the substantia nigra pars compacta were exceptional neurons expressing Pael receptors (Fig. 21 A to D).

As described above, we identified a putative seven-transmembrane domain protein (named the Pael receptor) that binds to Parkin, and elucidated its characteristics. Based on the following facts, we concluded that the protein is an *in vivo* original substrate protein of Parkin.

First, Parkin is thought to be involved in ERAD because Parkin expression is increased with BiP by UPR, and Parkin binds to UBC6 and UBC7, the endoplasmic reticulum-associated E2. Second, the Pael receptor is specifically ubiquitinated in the presence of UBC6 and UBC7 *in vitro* and in the Parkin-dependent ubiquitination pathway. Third, degradation of Pael receptors is promoted by causing the overexpression of Parkin within cells. Finally, Pael receptor proteins are accumulated in the detergent-insoluble fraction of AR-JP brain.

Recent findings have proposed an ER transmembrane model associated with the ubiquitin-proteasome system (Plemper, R. K. et al., (1999) Trends Biochem Sci 24, 266-270). Pael receptors are accumulated in the ER at an early stage of proteasome inhibition. This agrees with some recent reports that proteasome functions so as to drag proteins (to be transported back to the cytoplasm) out from the ER upon ERAD (Fig. 13) (Mayer, T. U. et al., (1998) Embo J 17, 3251-3257; Yu, H. et al., (1999) J Biol Chem 274, 36852-36858).

On the contrary, when proteasome inhibition was continued for a long time period, most cells came to have an apoptosis-like shape, curled up and shrank, so as to form intracellular aggregations (Fig. 14). Degradation by ubiquitination and proteasome during ERAD is a phenomenon that takes place in the cytoplasm, for which the retrograde transportation of misfolded proteins from the endoplasmic reticulum is a precondition. Thus, it is considered that the aggregation of Pael receptors in the cytoplasm corresponds to ubiquitinated insoluble Pael receptors detected by Western blotting (Fig. 10).

Based on the experimental results using culture cells, it seems that Parkin prevents the aggregation of Pael receptors that have been transported back from the endoplasmic reticulum to the cytoplasm by driving the degradation system so as to suppress cell death. Furthermore, Parkin may suppress endoplasmic reticulum stress by promoting the retrograde transportation of Pael receptors from the endoplasmic reticulum in cooperation with translocon complexes and proteasome complexes.

A pathological feature of AR-JP is a lack of Lewy bodies. This can be well explained by a hypothesis that Pael receptor accumulation is a factor that causes the onset of AR-JP. Pael receptors, the putative membrane protein, clearly stay in the endoplasmic reticulum when treated with lactacystin, the proteasome inhibitor, or UPR inducer. This suggests that folding of the Pael receptor is ineffective. When the quantity of misfolded proteins increases to too great an extent in the endoplasmic reticulum, causing an uncontrollable situation, cells (at least yeast cells) cope with the situation by activating using UPR the expression of many genes of such as molecular chaperones or ERAD-associated proteins (Travers, K. J. et al., (2000) Cell 101, 249-258; Friedlander, R. et al., (2000) Nat Cell Biol 2, 379-384). However, the ability of the endoplasmic reticulum to cope with misfolded proteins is limited. The endoplasmic reticulum seems to have a special apoptosis pathway against endoplasmic reticulum stresses such as a disturbance in calcium homeostasis within the lumen or the accumulation of misfolded proteins (Nakagawa, T. et al., (2000) Nature 403, 98-103; Urano, F. et al., (2000) Science 287, 664-666). Hence, neuronal cell death resulting from endoplasmic reticulum stress can be caused without the formation of aggregates. Similarly in AR-JP patients, an endoplasmic reticulum-specific apoptosis pathway may be initiated without the formation of intracellular aggregates that is caused by Pael receptor accumulation, so as to cause the onset of the disease.

Histological distribution of Pael receptors also agrees with dopamine neuron-selective neurodegeneration observed in AR-JP. Pael receptor mRNA is particularly highly expressed in the central nervous system. In the brain, Pael receptors are particularly expressed highly in the corpus callosum and substantia nigra (Donohue, P. J. et al, (1998) Brain Res Mol Brain Res 54, 152-160; Zeng, Z. et al., (1997) Biochem Biophys Res Commun 233, 559-567). In the present study, we studied the immunohistochemical distribution of Pael receptors in the mouse brain using anti-sera that specifically recognized Pael receptors (Fig. 20). Most Pael receptor-positive cells were CNPase-positive oligodendrocytes of the fiber bundle. Moreover, expression of Pael receptors was also observed in some of neurons such as those in the substantia nigra, hippocampus CA3 region or Purkinje cells (Fig. 21; data not shown).

In consideration of the fact that neurons are weaker against endoplasmic reticulum stress than oligodendrocytes, the cause of selective cell death in domapine neurons in AR-JP can be partially explained by the unique expression pattern of Pael receptors. The reason why only dopamine neurons among neurons expressing Pael receptors are selectively damaged is not well understood. This could be due to the fact that the expression level of Pael receptors in substantia nigra neurons is considerably high as determined by Northern blotting in past reports (Donohue, P. J. et al, (1998) Brain Res Mol Brain Res 54, 152-160; Zeng, Z. et al., (1997) Biochem Biophys Res Commun 233, 559-567).

Most recently, Zuscik et al have reported that the substantia nigra is also degenerated similarly to other regions in the brain of a transgenic mouse that over-expresses alpha 1B adrenalin receptors belonging to G protein-conjugated receptors, same as Pael receptors, and Parkinson's-disease-like symptoms are developed (Zuscik, M. J. et al., (2000) Nat Med 6, 1388-1394). Blocking of the signal transduction of alpha 1 adrenalin receptors partially improves the symptoms. However, since the effect of the improvement is insufficient, it is considered that endoplasmic reticulum stress resulting from misfolded proteins may cause substantia nigra degeneration also in the transgenic mouse. The substantia nigra seems to be particularly weak against stress induced by misfolded proteins, which is produced by ERAD, or when the protein quality control system of the endoplasmic reticulum is damaged.

In conclusion, we presented strong evidence that supports the idea that the accumulation of misfolded Pael receptor proteins causes AR-JP. In recent years, the accumulation of degenerated proteins is thought to be involved in the causes of many neurodegenerative diseases such as amyotrophic lateral sclerosis, Alzheimer disease, Parkinson's disease and polyglutamine disease (Julien, J. (2001) Cell 104, 581-591; Sherman, M. Y. et al., (2001) Neuron 29, 15-32). Pathological and biochemical evidence that is increasingly obtained from the studies on the above diseases presents the common etiological mechanism of neurodegenerative diseases in which the ubiquitin-proteasome pathway and damaged molecular chaperones are involved. AR-JP can now be added to the list of neurodegenerative diseases in which the accumulation of misfolded proteins is involved.

### Industrial Applicability

As clearly shown by the results in the examples, the Pael receptor of the present invention is a substrate of Parkin, and Pael receptor accumulation is a factor causing the onset of AR-JP. Hence, by the use of cells or transgenic animals expressing the Pael receptor of the present invention, the cause of Parkinson's disease can be elucidated, and a screening system using the Pael receptor quantity as an indicator can be obtained in order to develop a remedy for Parkinson's disease. Moreover, the Pael receptor gene can be a target of gene diagnosis as a risk factor of the onset of Parkinson's disease, and gene diagnosis can be performed using the whole or a part of the Pael receptor gene of the present invention. Furthermore, antibodies specific to the Pael receptor of the present invention can also be used for, for example, diagnosis and elucidation of the cause of Parkinson's disease.

All publications, patents and patent applications cited herein are incorporated herein by reference in their entirety. It is therefore to readily be understood by a person skilled in the art that numerous modifications and variations of the present invention are possible within the scope of the invention without departing from the technical idea and the scope of the invention as described in the appended claims. The present invention is intended to encompass such modifications and variations.

## Claims

1. A human Pael receptor, for screening for a remedy for Parkinson's disease.

2. The human Pael receptor of claim 1, wherein Parkinson's disease is autosomal recessive hereditary Parkinson's disease.

3. An animal cell for screening for a remedy for Parkinson's disease, having a foreign DNA encoding a human Pael receptor or a DNA containing a variant of the DNA incorporated therein.

4. The animal cell of claim 3, wherein the foreign DNA encoding a human Pael receptor is represented by SEQ ID NO: 1.

5. The animal cell of claim 3 or 4, further having the Parkin gene or a variant of the Parkin gene.

6. The animal cell of claim 5, further having a gene encoding at least one protein selected from the group consisting of endothelin receptor type A, endothelin receptor type B, UBC6 and UBC7.

7. The animal cell of any one of claims 3 to 6, wherein the cell is an HEK293T cell or SH-SY5Y cell.

8. The animal cell of any one of claims 3 to 7, wherein Parkinson's disease is autosomal recessive hereditary Parkinson's disease.

9. A non-human mammal, having a foreign DNA encoding a human Pael receptor, or a DNA containing a variant of the DNA incorporated therein.

10. The non-human mammal of claim 9, wherein the foreign DNA encoding a human Pael receptor is represented by SEQ ID NO: 1.

11. The non-human mammal of claim 9 or 10, further having the Parkin gene or a variant of the Parkin gene.

12. The non-human mammal of claim 11, further having a gene encoding at least one protein selected from the group consisting of endothelin receptor type A, endothelin receptor type B, UBC6 and UBC7.

13. The animal of any one of claims 9 to 12 above, wherein the non-human mammal is a rodent.

14. The animal of claim 13, wherein the rodent is a mouse.

15. The non-human mammal of any one of claims 9 to 14, which is a mammal obtained by ontogenesis of a totipotent cell having DNA encoding a human Pael receptor or its variant introduced therein, or the progeny of such mammal, and which carries the transgene in the somatic cell chromosome.

16. A cell isolated from the animal of any one of claims 9 to 15.

17. A method of screening for a remedy for Parkinson's disease, which comprises causing the human Pael receptor of claim 1 or 2 to come into contact with a candidate remedy for Parkinson's disease, and using the Pael receptor quantity in animal cells as an indicator.

18. A method of screening for a remedy for Parkinson's disease, which comprises causing the animal cells of any one of claims 3 to 8 and claim 16 to come into contact with a candidate remedy for Parkinson's disease, and using the Pael receptor quantity in the animal cells as an indicator.

19. A method of screening for a remedy for Parkinson's disease, which comprises administering a candidate remedy for Parkinson's disease to the animal of any one of claims 9 to 15, and using the Pael receptor quantity in the cell of the animal as an indicator.

20. The method of any one of claims 17 to 19, wherein Parkinson's disease is autosomal recessive hereditary Parkinson's disease.

21. A probe, consisting of the whole or a part of a DNA encoding a human Pael receptor for detecting a risk factor for the onset of Parkinson's disease.

22. The probe of claim 21, wherein Parkinson's disease is autosomal recessive hereditary Parkinson's disease.

23. A method of detecting a risk factor for the onset of Parkinson's disease, using the probe of claim 21 or 22.

24. The method of claim 23, wherein Parkinson's disease is autosomal recessive hereditary Parkinson's disease.

25. An antibody recognizing the Pael receptor, which reacts with the Pael receptor but does not react with ETBR-LP-2 which is a Pael receptor homolog.

26. The antibody of claim 25, which further does not react with endothelin receptor type A or endothelin receptor type B, which is a Pael receptor homolog.

27. The antibody of claim 25 or 26, which is a monoclonal antibody.


SEQUENCE LISTING

<110> RIKEN

<120> Pael receptor, a cell and animal which express the Pael receptor,
and a method for screening a medication for Parkinson disease

<130> PH-1575-PCT

<140>
<141>

<150> JP 2001/197185
<151> 2001-06-28

<160> 8

<170> PatentIn Ver. 2.1

<210> 1
<211> 1842
<212> DNA
<213> Homo sapiens

<220>
<221> CDS
<222> (1)..(1839)

<400> 1
atg cga gcc ccg ggc gcg ctt ctc gcc cgc atg tcg cgg cta ctg ctt 48
ctg cta ctg ctc aag gtg tct gcc tct tct gcc ctc ggg gtc gcc cct 96
gcg tcc aga aac gaa act tgt ctg ggg gag agc tgt gca cct aca gtg 144
atc cag cgc cgc ggc agg gac gcc tgg gga ccg gga aat tct gca aga 192
gac gtt ctg cga gcc cga gca ccc agg gag gag cag ggg gca gcg ttt 240
ctt gcg gga ccc tcc tgg gac ctg ccg gcg gcc ccg gac cgt gac ccg 288
gct gca ggc aga ggg gcg gag gcg tcg aca gcc gga ccc ccg gga cct 336
cca acc agg cca cct gtc ccc tgg agg tgg aaa ggt gct cgg ggt cag 384
gag cct tct gaa act ttg ggg aga ggg aac ccc acg gcc ctc cag ctc 432
ttc ctt cag atc tca gag gag gaa gag aag ggt ccc aga ggc gct gtc 480
att tcc ggg cgt agc cag gag cag agt gtg aag aca gtc ccc gga gcc 528
agc gat ctt ttt tac tgt cca agg aga gcc ggg aaa ctc cag ggt tcc 576
cac cac aag ccc ctg tcc aag acg gcc aat gga ctg gcg ggg cac gaa 624
ggg tgg aca att gca ctc ccg ggc cgg gcg ctg gcc cag aat gga tcc 672
ttg ggt gaa gga atc cat gat cct ggg ggt ccc cgc cgg gga aac agc 720
acg aac cgg cgt gtg aga ctg aag aac ccc ttc tac ccg ctg acc cag 768
gag tcc tat gga gcc tac gcg gtc atg tgt ctg tcc gtg gtg atc ttc 816
ggg acc ggc atc att ggc aac ctg gcg gtg atg tgc atc gtg tgc cac 864
aac tac tac atg cgg agc atc tcc aac tcc ctc ttg gcc aac ctg gtc 912
ttc tgg gac ttt ctc atc atc ttc ttc tgc ctt ccg ctg gtc atc ttc 960
cac gag ctg acc aag aag tgg ctg gtg gag gac ttc tcc tgc aag atc 1008
gtg ccc tat ata gag gtc gct tct ctg gga gtc acc act ttc acc tta 1056
tgt gct ctg tgc ata gac cgc ttc cgt gct gcc acc aac gta cag atg 1104
tac tac gaa atg atc gaa aac tgt tcc tca aca act gcc aaa ctt gct 1152
gtt ata tgg gtg gga gct cta ttg tta gca ctt cca gaa gtt gtt ctc 1200
cgc cag ctg agc aag gag gat ttg ggg ttt agt ggc cga gct ccg gca 1248
gaa agg tgc att att aag atc tct cct gat tta cca gac acc atc tat 1296
gtt cta gcc ctc acc tac gac agt gcg aga ctg tgg tgg tat ttt ggc 1344
tgt tac ttt tgt ttg ccc acg ctt ttc acc atc acc tgc tct cta gtg 1392
act gcg agg aaa atc cgc aaa gca gag aaa gcc tgt acc cga ggg aat 1440
aaa cgg cag att caa cta gag agt cag atg aac tgt aca gta gtg gca 1488
ctg acc att tta tat gga ttg ggc att att cct gaa aat atc tgc aac 1536
att gtt act gcc tac atg gct aca ggg gtt tca cag cag aca atg gac 1584
ctc ctt aat atc atc agc cag ttc ctt ttg ttc ttt aag tcc tgt gtc 1632
acc cca gtc ctc ctt ttc tgt ctc tgc aaa ccc ttc agt cgg gcc ttc 1680
atg gag tgc tgc tgc tgt tgc tgt gag gaa tgc att cag aag tct tca 1728
acg gtg acc agt gat gac aat gac aac gag tac acc acg gaa ctc gaa 1776
ctc tcg cct ttc agt gcc ata cgc cgt gaa atg tcc act ttt gct tct 1824
gtc gga act cat tgc tga 1842

<210> 2
<211> 613
<212> PRT
<213> Homo sapiens

<400> 2

<210> 3
<211> 20
<212> DNA
<213> Artificial Sequence

<220>
<223> Description of Artificial Sequence:Synthetic DNA

<400> 3
cctccagctc ttccttcaga                                                20

<210> 4
<211> 22
<212> DNA
<213> Artificial Sequence

<220>

<223> Description of Artificial Sequence:Synthetic DNA

<400> 4
tttctgccgg agctctcggc ca                                                22

<210> 5
<211> 21
<212> DNA
<213> Artificial Sequence

<220>
<223> Description of Artificial Sequence:Synthetic DNA

<400> 5
ggaggcgacg accccagaaa c                                                 21

<210> 6
<211> 21
<212> DNA
<213> Artificial Sequence

<220>
<223> Description of Artificial Sequence:Synthetic DNA

<400> 6
gggacagcca gccacacaag g                                                 21

<210> 7
<211> 20
<212> DNA
<213> Artificial Sequence

<220>
<223> Description of Artificial Sequence:Synthetic DNA

<400> 7
caaggccaac cgcgagaaga                                                    20



<210> 8
<211> 20

<212> DNA
<213> Artificial Sequence

<220>
<223> Description of Artificial Sequence:Synthetic DNA

<400> 8
ggaaggctgg aagagtgcct                                                    20